# EUROPÄISCHE PATENTSCHRIFT

(11) **EP 4 633 545 B1**
(45) Veröffentlichungstag und Bekanntmachung des Hinweises auf die Patenterteilung: **07.10.2026**
(21) Anmeldenummer: 23832718.3
(22) Anmeldetag: 13.12.2023
(51) Int. Cl.: A61F 2/64, A61F 2/66, A61F 2/68, A61F 2/76, A61F 2/70

(54) **ORTHOPÄDIETECHNISCHE EINRICHTUNG UND VERFAHREN ZU DEREN STEUERUNG**
ORTHOPAEDIC DEVICE AND METHOD FOR CONTROLLING SAME
DISPOSITIF ORTHOPÉDIQUE ET PROCÉDÉ POUR EN ASSURER LA COMMANDE

(30) Priorität: 15.12.2022 DE 102022133462
(43) Veröffentlichungstag der Anmeldung: 22.10.2025
(73) Patentinhaber: Otto Bock Healthcare Products GmbH, 1110 Wien (AT)
(72) Erfinder: SEIFERT, Dirk, 1110 Wien (AT)
(74) Vertreter: Gramm, Lins & Partner Patent- und Rechtsanwälte PartGmbB
(86) Internationale Anmeldenummer: PCT/EP2023/085624
(87) Internationale Veröffentlichungsnummer: WO 2024/126590

(56) Entgegenhaltungen:
- US-B2- 8 453 340

## Beschreibung

Die Erfindung betrifft ein Verfahren zur Steuerung einer orthopädietechnischen Einrichtung der unteren Extremität mit einem Oberteil und einem Unterteil, die gelenkig um zumindest eine Schwenkachse miteinander unter Ausbildung eines Gelenkes aneinander gelagert sind und mit zumindest einem Aktuator, der mit einer Steuerungseinrichtung gekoppelt ist, die auf der Grundlage von Sensordaten zumindest eines mit der Steuerungseinrichtung gekoppelten Sensors den Aktuator aktiviert oder deaktiviert, um einen Verschwenkungswiderstand oder eine Relativbewegung des Oberteils zu dem Unterteil zu beeinflussen und eine solche orthopädietechnische Einrichtung, insbesondere zur Durchführung des Verfahrens.

Unter orthopädietechnischen Einrichtungen der unteren Extremität werden insbesondere Orthesen und Prothesen verstanden. Orthesen sind orthopädietechnische Hilfsmittel, die an einer existierenden Gliedmaße angelegt werden und die Bewegungen führen, einschränken oder unterstützen. Zwischen gelenkig miteinander verbundenen Komponenten können Antriebe, Aktuatoren und/oder Widerstandseinrichtungen, die über einen Aktuator verstellt oder eingestellt werden können, angeordnet sein. Die Verstellung kann auf Basis von Sensordaten, die einer Datenverarbeitungseinrichtung übermittelt werden, erfolgen. Unter Orthesen werden im Rahmen dieser Anmeldung auch Exoskelette verstanden, die an dem Körper eines Patienten angelegt werden und eine äußere Stützstruktur bilden, insbesondere um die Bewegungen einer nutzenden Person zu führen und zu beeinflussen, z.B. durch Antriebe zu unterstützen oder über Widerstandseinrichtungen zu bremsen. Orthesen und Exoskelette als deren Spezialfälle können neben der Unterstützung der täglichen Verrichtungen auch zu Trainingszwecken oder zu therapeutischen Zwecken verwendet und eingesetzt werden.

Prothesen ersetzen nicht oder nicht mehr vorhandene Gliedmaßen. Die einfachsten Prothesenkomponenten haben eine rein kosmetische Funktion oder vervollständigen eine Gliedmaße, beispielsweise indem ein distales Fingerglied ersetzt wird. Im Laufe der Zeit wurden die Prothesen komplexer, mehrere Prothesenkomponenten wurden aneinander angeordnet und befestigt und beispielsweise über Gelenke miteinander verbunden. Komplexe mechanische Antriebsvorrichtungen wurden entwickelt, um beispielsweise Prothesenhände oder Prothesenfüße zu bewegen. Hydraulische oder andere Dämpfereinrichtungen oder Widerstandseinrichtungen wurden an Gelenken angeordnet, um das Verhalten von Prothesenkomponenten und Prothesensystemen zu modifizieren, um einen möglichst natürlichen Bewegungsablauf zu ermöglichen. Zur Unterstützung von Bewegungen wurden Antriebe in Prothesenkomponenten integriert, sodass aktive Prothesen entstanden sind. Weiterhin wurden Sensoren an Prothesenkomponenten oder einer die Prothese nutzenden Person angeordnet, um das gegenwärtige Bewegungsverhalten oder die gegenwärtigen Positionen oder Stellungen von Prothesenkomponenten zueinander zu erfassen und das zukünftige Bewegungsverhalten abzuschätzen und um Einstellungen an Widerstandseinrichtungen und/oder Antrieben zu verändern. Dadurch sind hochkomplexe Prothesensysteme mit mehreren, aneinander angeordneten Prothesenkomponenten entstanden, die eine Vielzahl von mechanischen, elektrischen und mechatronischen Komponenten aufweisen.

Ein Prothesensystem der unteren Extremität kann insbesondere einen Oberschenkelschaft aufweisen, an dem an dessen distalen Ende ein Prothesenkniegelenk, ein Prothesen-Unterschenkel und ein Prothesenfuß befestigt sind. Ein solches Prothesensystem weist beispielsweise zwei oder mehr Gelenke auf, die jeweils mit Widerstandseinrichtungen und/oder Antrieben bzw. Aktuatoren versehen sein können.

Aus der EP 2 816 979 B1 ist ein Verfahren zur Steuerung eines künstlichen Orthesenkniegelenkes oder Prothesenkniegelenkes bekannt, bei dem auf der Grundlage der Erfassung eines Absolutwinkels einer Unterschenkelkomponente der Beugewiderstand verändert wird. Der ermittelte Absolutwinkel der Unterschenkelkomponente wird mit einem Schwellwert verglichen, wird der Schwellenwert erreicht oder überschritten, wird der Beugewiderstand verändert.

Aus der EP 2 649 968 B1 ist ein Verfahren zur Steuerung eines orthopädischen Fußteils mit einem Knöchelgelenk bekannt, bei dem am Knöchelgelenk auftretende Drehmomente, der Knöchelwinkel sowie der Absolutwinkel eines Fußteils bezogen auf die Vertikale bestimmt werden. In Abhängigkeit von den gemessenen Werten wird das Abrollen des Fußes in der Standphase, die Stellung des Fußteils in der Schwungphase sowie die Stellung und Bewegbarkeit des Fußteils während des Stehens mittels einer Dämpfungsanordnung gesteuert.

Zur Umschaltung zwischen verschiedenen Betriebsmodi ist es zudem bekannt, dass orthopädietechnische Einrichtungen der unteren Extremität besonders belastet werden, um einen Sondermodus einzustellen. Ein mehrfaches, rhythmisches Belasten in einem bestimmten Zeitraum in einer bestimmten Belastungsrichtung wird als Schaltsignal gewertet, um dann, beispielsweise für das Treppen aufwärts Gehen, besondere Programme zu aktivieren. Das bewusste Umschalten der Betriebsmodi erfordert eine hohe Aufmerksamkeit durch die nutzende Person der orthopädietechnischen Einrichtung.

Die US 8 453 340 B2 betrifft ein Sensormodul mit einer IMU und gegebenenfalls weiteren Sensoren, die mit einer CPU in Verbindung stehen und dieser Eingangssignale von zumindest einem Orientierungssensor oder IMU übermitteln. Die CPU gibt einen entsprechenden Wert für den Rollwinkel, Nickwinkel und Gierwinkel über eine Schnittstelle an eine Steuerung, um beispielsweise eine Armprothese mit Antrieben zu steuern. Auf der Grundlage der unterschiedlichen Orientierungen des Sensormoduls wird die Prothese entsprechend angesteuert und bewegt.

Aufgabe der vorliegenden Erfindung ist es, ein Verfahren und eine orthopädietechnische Einrichtung bereitzustellen, mit denen nutzende Personen von orthopädietechnischen Einrichtungen der unteren Extremität Aktivitäten des täglichen Lebens einfacher ausführen können und mit denen es möglich ist, eine vielseitigere Nutzung der orthopädietechnischen Einrichtung durchzuführen.

Erfindungsgemäß wird diese Aufgabe durch ein Verfahren mit den Merkmalen des Hauptanspruches gelöst und durch eine orthopädietechnische Einrichtung mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Verfahren zur Steuerung einer orthopädietechnischen Einrichtung der unteren Extremität mit einem proximalen Oberteil und einem distalen Unterteil, die gelenkig um zumindest eine Schwenkachse miteinander unter Ausbildung eines Gelenkes aneinander gelagert sind, mit Einrichtungen zum Festlegen der orthopädietechnischen Einrichtung an einer Gliedmaße und zumindest einem Aktuator, der mit einer Steuerungseinrichtung gekoppelt ist, die auf der Grundlage von Sensordaten zumindest eines mit der Steuerungseinrichtung gekoppelten Sensors den Aktuator aktiviert oder deaktiviert, um einen Verschwenkungswiderstand und/oder eine Relativbewegung des Oberteils zu dem Unterteil oder zweier Bauteile der orthopädietechnischen Einrichtung zueinander zu beeinflussen, sieht vor, dass über die Sensordaten eine Orientierung und/oder Orientierungsänderung um die Längsachse der Längserstreckung in proximal-distal Richtung der orthopädietechnischen Einrichtung und/oder einer kontralateralen Gliedmaße erfasst werden und dass auf der Grundlage der Orientierung und/oder Orientierungsänderung um die Längsachse der Aktuator aktiviert, deaktiviert oder ein Sollwert für den Aktuator moduliert wird. Während in dem Stand der Technik die Bewegungen in der Sagittalebene ausgewertet und zur Modifikation von Widerständen oder Antrieben eingesetzt werden, wird erfindungsgemäß eine Verlagerung um eine Längsachse der Längserstreckung der orthopädietechnischen Einrichtung oder der kontralateralen Gliedmaße oder um beide Längsachsen erfasst und dazu verwendet, die Widerstände oder Antriebe der orthopädietechnischen Einrichtung entsprechend zu beeinflussen. Verlagerungen um die Längsachse finden insbesondere bei der Ausführung von Drehungen statt, beispielsweise beim Gehen um eine Kurve, abrupten Richtungsänderungen, sportlichen Aktivitäten und dergleichen. Mit der Berücksichtigung solcher Bewegungen ist es möglich, die orthopädietechnische Einrichtung mikroprozessorgesteuert an die jeweiligen Bewegungen und Situationen, die von geradlinigen Bewegungen in der Sagittalebene abweichen, anzupassen, insbesondere in der Schwungphase z.B. des Kurvengehens. Auch bei orthopädietechnischen Einrichtungen der oberen Extremität sind solche Steuerungsvorgänge vorteilhaft und sinnvoll, um auf die Vielzahl von möglichen Bewegungen und Bewegungsmustern bei der Verrichtung der alltäglichen Tätigkeiten eingehen zu können und um keine unnötigen Einschränkungen hinnehmen zu müssen. Bei einer Orientierungsänderung um die Längsachse der Längserstreckung der versorgten Seite, der kontralateralen Gliedmaße und/oder des Körpers wird ein Flexionswiderstand in der Standphase der versorgten Seite, insbesondere in der terminalen Standphase, nicht reduziert und/oder eine Flexion nicht unterstützt. Alternativ oder ergänzend erfolgen eine geringere Reduktion des Flexionswiderstands und/oder eine geringere Unterstützung der Flexionsbewegung als beim Geradeausgehen. Beim geradeaus Gehen wird in einem künstlichen Kniegelenk typischerweise ein Flexionswiderstand in der terminalen Standphase reduziert oder die Flexion freigegeben, um ein leichtes Einbeugen in der Schwungphaseneinleitung zu ermöglichen. Bei einem aktiven Kniegelenk wird die Flexionsbewegung aktiv unterstützt, um ein besonders leichtes Einbeugen und einen ausreichend hohen Kniebeugewinkel zu erzielen. In einem aktiven Knöchelgelenk wird in der terminalen Standphase ein Plantarflexionsmoment generiert, um den Fuß und damit auch den Körper in Gehrichtung nach vorne abzustoßen. Bei einer Rotation mit der versorgten Seite als Standbein kann es vorteilhaft sein, den Beugewiderstand im Kniegelenk nicht oder nur in einem geringeren Ausmaß zu reduzieren, um ein ungewünschtes oder unerwartetes Einbeugen zu verhindern. Der Beugewiderstand wird in einer solchen Ausgestaltung bei Detektion einer Rotation auf einem typischen Standphasen-Niveau belassen oder nur teilweise reduziert. Bei einem aktiven Kniegelenk wird keine Beugebewegung eingeleitet oder unterstützt oder die Unterstützung der Beugebewegung reduziert. Bei einem aktiven Fuß wird die aktive Plantarflexion nicht eingeleitet oder weniger stark unterstützt als beim geradeaus Gehen. Insbesondere bei Versorgung von Personen mit einem erhöhten Sicherheitsbedürfnis oder einer geminderten Koordinationsfähigkeit kann dies einen Vorteil gegenüber bestehenden Systemen darstellen. Insbesondere bei Richtungswechseln und Drehbewegungen auf engem Raum kann eine unerwartete Reduktion eines Flexionswiderstands oder eine unerwartete Einleitung einer Flexionsbewegung zu Unsicherheit, dem Gleichgewichtsverlust oder einem Sturz führen.

Eine Weiterbildung sieht vor, dass während der Nutzung der orthopädietechnischen Einrichtung im angelegten Zustand die Sensordaten ermittelt werden und der Aktuator aktiviert, deaktiviert oder der Sollwert für den jeweiligen Aktuator moduliert wird. Damit ist es möglich, den Verschwenkungswiderstand und/oder oder eine Relativbewegung des Oberteils zu dem Unterteil angepasst an die jeweilige Bewegung und die jeweilige Bewegungssituation zu beeinflussen.

Die Orientierung oder die Verlagerung der orthopädietechnischen Einrichtung oder von Teilen der orthopädietechnischen Einrichtung wird in einer Ausgestaltung über einen Raumlagesensor, zumindest eine IMU (inertial measurement unit) und/oder zumindest einen Winkelsensor erfasst und ermittelt. Die Winkelsensoren oder der Winkelsensor erfasst die Stellung von Oberteil zu Unterteil, einzelner Komponenten zueinander oder auch der Komponenten relativ zu einem Körperteil oder einem anderen Bezugselement an der nutzenden Person und ermöglichen die Feststellung der Orientierung der gesamten orthopädietechnischen Einrichtung, mehrerer Teile davon oder auch nur eines Teils davon in Hinblick auf die jeweilige Position, Orientierung und Bewegung um eine Längsachse entlang der Längserstreckung entweder der orthopädietechnischen Einrichtung oder der kontralateralen Gliedmaße oder des Rumpfes. Über einen Raumlagesensor oder eine IMU können unmittelbar die Orientierungen von Komponenten bzw. der gesamten orthopädietechnischen Einrichtung und sofern notwendig die Orientierung der Komponenten zueinander bestimmt werden. Eine IMU kann so ausgeführt sein, dass ein Magnetfeld, insbesondere das Erdmagnetfeld, in einer Richtung oder mehreren Richtungen erfasst und für die Berechnung der Orientierung verwendet wird. Damit ist es möglich, die Orientierung einer Komponente oder mehrerer Komponenten gegenüber dem Magnetfeld zu bestimmen. Nach der Auswertung werden die Orientierungen der Steuerung zur Aktivierung, Deaktivierung oder Modulierung eines Sollwertes des Aktuators zur Veränderung des Verschwenkungswiderstandes und/oder der Relativbewegung entsprechende Befehle übermittelt. Ein Relativwinkel zwischen zwei Komponenten kann aus den jeweiligen Absolutwinkeln bestimmt werden, beispielsweise durch zwei IMUs. Aus dem Absolutwinkel oder Raumwinkel einer Komponente und den Relativwinkeln zu anderen Komponenten können deren Absolutwinkel bestimmt werden.

In einer Weiterbildung werden Kräfte, Momente und/oder Beschleunigungen der gesamten orthopädietechnischen Einrichtung und/oder deren Komponenten über Sensoren erfasst und der Steuerung ebenfalls zugrunde gelegt. In Verbindung mit der Erfassung von Verschwenkungen und/oder Verlagerungen, z.B. innerhalb der Frontalebene und/oder in der Sagittalebene, werden zusätzliche Parameter zur Steuerung des Aktuators herangezogen. Ein Wegfall von Axialkräften zeigt beispielsweise, dass die orthopädietechnische Einrichtung sich in einer Schwungphase befindet. Unterschiedliche Kraftverteilungen oder Einleitungen von Momenten um Schwenkachsen ermöglichen eine Bestimmung von Bewegungen, Bewegungsänderungen, Zuständen und wahrscheinlichen zukünftigen Bewegungen oder Belastungen, sodass auf Grundlage der Kräfte, Momente und/oder Beschleunigungen, insbesondere in Verbindung mit Daten zur Orientierung und/oder Verlagerung um die Längsachse der Längserstreckung in proximal-distal-Richtung, der Aktuator mit entsprechenden Befehlen versorgt wird. Kräfte und Momente können über eine Verformung, Verschiebung, Verkippung und/oder eine Kombination aus diesen bestimmt werden. Zum Beispiel kann aus der Verformung eines elastischen oder kompressiblen Körpers und/oder aus einer daraus resultierenden Verkippung oder Verschiebung auf eine einwirkende Kraft und/oder ein Moment geschlossen werden. Über die Verschiebungs- oder Verformungsrate eines Dämpfers oder viskosen Elements kann ebenfalls auf eine Kraft und/oder ein Moment geschlossen werden.

In einer Weiterbildung wird zumindest eine Orientierung und/oder Orientierungsänderung um die Längsachse der orthopädietechnischen Einrichtung und/oder einer kontralateralen Gliedmaße und/oder des Körpers auf Basis von Sensordaten und einem Modell geschätzt oder errechnet. Über Modelle können Größen errechnet oder geschätzt werden, die messtechnisch nicht oder nur schwer zugänglich sind. Über mechanische Modelle können mittels Bewegungsgleichungen aus Kräften und Momenten Bewegungen errechnet werden. Über Filter, zum Beispiel Kalman-Filter, können Zustandsgrößen kontinuierlich aus anderen Sensordaten geschätzt werden. Mittels Algorithmen der künstlichen Intelligenz, wie neuronalen Netzen, rekursiven neuronalen Netzen, Convolutional-Neural-Networks, State-Vector-Machines, linearer Diskriminanzanalyse, K-Means oder Regression können Zustandsgrößen, wie die Orientierung und/oder Orientierungsänderung, aus anderen Sensordaten geschätzt werden. Die Schätzung und/oder Berechnung kann kontinuierlich oder zu bestimmten Zeitpunkten erfolgen.

In einer Weiterbildung wird die Orientierung und/oder Orientierungsänderung um die Längsachse der orthopädietechnischen Einrichtung und/oder einer kontralateralen Gliedmaße und/oder des Körpers zumindest einer Komponente mittels einer Umgebungssensorik, insbesondere mittels Erfassung elektromagnetischer Strahlung, gegenüber einer Referenz ermittelt. Die Referenz kann die Umgebung und/oder eine kontralaterale Gliedmaße sein. Die elektromagnetische Strahlung kann von dem orthopädietechnischen Hilfsmittel für die Messung ausgesendet werden. Über eine oder mehrere Kameras, Tiefenbildkameras, Radar- und/oder Lidar-Sensoren und dergleichen kann die Umgebung erfasst und die Orientierung und/oder Orientierungsänderung bestimmt werden. Über Laufzeitmessungen von elektromagnetischer Strahlung, aber auch mittels Sonar, kann die Distanz zu einem oder mehreren Objekten der Umgebung und damit die Orientierung und/oder Orientierungsänderung bestimmt werden. Mittels globaler Navigationssysteme und/oder Indoor-Navigation, beispielsweise mittels Beacons oder WLAN, kann die Orientierung und/oder Orientierungsänderung ermittelt werden. Es ist auch möglich, dass die Orientierung und/oder Orientierungsänderung der orthopädietechnischen Hilfseinrichtung über ein externes System erfasst wird, beispielsweise über eine oder mehrere Kameras, Tiefenbildkameras, Radar- und/oder Lidar-Sensoren, und die Information an die Hilfseinrichtung übertragen wird. Die Orientierung und/oder Orientierungsänderung kann über zeitlich aufeinanderfolgende Positionen ermittelt werden, welche über die Umgebungssensorik bestimmt werden.

In einer Ausgestaltung ist die orthopädietechnische Einrichtung als Prothese oder Orthese ausgebildet und weist ein künstliches Kniegelenk und/oder Knöchelgelenk auf, wobei jedem Gelenk ein Aktuator zugeordnet ist. Sind sowohl ein Kniegelenk als auch ein Knöchelgelenk vorhanden, können zwei Aktuatoren vorhanden sein, um individuell und unabhängig voneinander einen Verschwenkungswiderstand um das jeweilige Gelenk zu verändern bzw. eine Relativbewegung zwischen dem jeweiligen Oberteil und dem jeweiligen Unterteil einzuleiten oder zu beeinflussen. Es besteht auch die Möglichkeit, dass nur ein einziger Aktuator zwei Gelenken zugeordnet ist, über den eine entsprechende Vergrößerung oder Verkleinerung eines Verschwenkungswiderstandes erreicht oder eine Verlagerung von Oberteil zu Unterteil bewirkt wird. Es können auch mehr als zwei Gelenke und ein oder mehrere Aktuatoren angeordnet sein und gesteuert werden.

In einer Weiterbildung wird der zumindest eine Sensor an der orthopädietechnischen Einrichtung, der kontralateralen Gliedmaße oder dem Rumpf der nutzenden Person angeordnet oder es werden mehrere Sensoren an der orthopädietechnischen Einrichtung, der kontralateralen Gliedmaße und/ oder dem Rumpf der nutzenden Person angeordnet. Über diesen Sensor bzw. die Sensoren werden Sensordaten ermittelt, die dann der weiteren Steuerung des Aktuators zugrunde gelegt werden. Durch die Anordnung von Sensoren ist es möglich, nicht nur die absolute Rotation der versorgten Seite um die Längsachse zu erfassen und zu steuern, sondern darüber hinaus oder alternativ die Rotation relativ zu dem Körper zu berücksichtigen. Insbesondere die Erfassung der relativen Bewegung und Stellung der versorgten Seite zu dem übrigen Körper erfolgt bevorzugt über mehrere Sensoren, da aus den verschiedenen Sensorwerten einfach die Relativverlagerungen und Relativdrehungen abgeleitet werden können. Insbesondere die IMUs erlauben eine vergleichsweise genaue und einfache sowie preiswerte Ermittlung von Bewegungen und Positionen im Raum in verschiedenen Ebenen und um verschiedene Achsen, sodass durch die Auswertung deren Daten einfach auf die vorhandene Bewegung geschlossen werden kann.

In einer Ausgestaltung ist vorgesehen, dass bei einer Rotation der versorgten Seite um die Längsachse der Längserstreckung der kontralateralen Gliedmaße in der Schwungphase der orthopädietechnischen Einrichtung eine Vergrößerung eines Flexionswiderstandes, insbesondere gegenüber dem geradeaus Gehen, eingeleitet wird. Alternativ oder ergänzend wird eine Verringerung eines Extensionswiderstandes eingeleitet oder eine Extension aktiv eingeleitet oder unterstützt oder eine bereits vorhandene Unterstützung erhöht. Im Gegensatz zu reinen Bewegungen in der Sagittalebene können die Bewegungen bei Rotationen um die Längsachse der Längserstreckung der kontralateralen Gliedmaße, insbesondere eines Beines, wesentlich länger dauern, sodass die üblichen Steuerungsmechanismen nicht oder nicht so effektiv anwendbar sind. Beim Gehen um eine Kurve kann es vorkommen, dass aufgrund der vom Gehen in der Ebene abweichenden Zeiträume das Kniegelenk nicht rechtzeitig vor dem Aufsetzen in die Streckung kommt. Dies tritt vor allem beim Gehen um die Kurve auf, bei dem in der Schwungphase der versorgten Seite eine Innenrotation um das kontralaterale Standbein ausgeführt wird. Eine solche Bewegung wird beispielsweise bei einem Richtungswechsel ausgeführt. Bei dem Vorliegen einer solchen Rotation ist es vorteilhaft, bei einem passiven Kniegelenk den Flexionswiderstand in der Schwungphase zu erhöhen, um den maximalen Kniebeugewinkel zu reduzieren. Dies führt zu einer verkürzten Pendelbewegung verglichen mit dem geradeaus Gehen in der Ebene. Energie, die im Vergleich zum geradeaus Gehen zusätzlich durch die nutzende Person im Zuge der Rotation in das System eingebracht wird, kann durch erhöhte Widerstände und/oder länger wirkende Widerstände dissipiert werden. Ein Streckwiderstand kann gegenüber dem geradeaus Gehen reduziert werden, um eine möglichst schnelle Streckung zu gewährleisten. Ist ein aktives Kniegelenk mit einer motorischen Unterstützung oder einer anderweitigen Freigabe einer gespeicherten Energiemenge vorgesehen, kann die Extension aktiv unterstützt werden und insbesondere stärker und/oder früher als beim geradeaus Gehen unterstützt werden. Dadurch ist es möglich, sowohl die Flexionsbewegung als auch die Extensionsbewegung so zu steuern, dass sie mit der Rotationsbewegung um das Standbein korrespondiert. So kann die Extension eingeleitet werden, wenn eine Verringerung der Rotationsbewegung oder eine Verlangsamung der Rotationsbewegung festgestellt wird.

Eine Weiterbildung des Verfahrens sieht vor, dass bei einer Rotation der versorgten Seite um die Längsachse der Längserstreckung der kontralateralen Gliedmaße in der Schwungphase der orthopädietechnischen Einrichtung eine Hüftbeugung unterstützt wird, beispielsweise über einen Motor oder eine Freigabe einer Energiespeichereinrichtung wie einer Feder oder eines Pneumatikspeichers. Alternativ oder ergänzend kann ein Hüftflexionswiderstand verringert werden, sodass eine Hüftflexion stärker oder schneller eingeleitet werden kann, wodurch eine Anpassung der Widerstände oder Unterstützungsraten an die jeweilige Rotationsbewegung erfolgt.

**In** einer Weiterbildung wird bei einer Rotation der versorgten Seite um die Längsachse der Längserstreckung der versorgten Seite in der Standphase der Flexionswiderstand erhöht, eine stattfindende oder vorhandene Einbeugung verringert und/oder eine Einbeugung oder weitere Einbeugung verhindert. Insbesondere kann ein Widerstand eines Knöchelgelenkes in Dorsalflexion gegenüber dem geradeaus Gehen erhöht werden, ggf. bis zu einer Sperre, alternativ kann auch eine Plantarflexion unterstützt werden. Es kann auch der Flexionswiderstand des Kniegelenkes gegenüber dem geradeaus Gehen erhöht werden, eine Beugebewegung abgestoppt werden und/oder eine Extensionsbewegung im Knie unterstützt werden. Es können sowohl die zeitlichen Verläufe als auch die Höhe der Widerstände und/oder Unterstützungsmomente angepasst werden. Mit einer solchen Erhöhung des Widerstandes oder einer Extension oder Sperrung einer weiteren Flexion ist es möglich, bei einer Rotation der versorgten Seite als Standbein die Ausführung der Drehbewegung zu erleichtern und ein ungewolltes Einbeugen zu verhindern. Dies erhöht die Stabilität und Sicherheit für die nutzende Person der orthopädietechnischen Einrichtung. Eine solche Erhöhung des Flexionswiderstandes bzw. Extension kann beispielsweise in dem Knöchelgelenk, Kniegelenk und/oder in dem Hüftgelenk stattfinden.

Die Schwungphase in einer Rotationsbewegung kann länger dauern als beim geradeaus Gehen. Dies ist vor allem dann der Fall, wenn es sich um eine Rotation um einen großen Winkel handelt, zum Beispiel ein Umdrehen mit einer Rotation um 180°, aber auch ganze bis mehrfache Drehungen um die Körperachse, die in speziellen Situationen auftreten können. In diesem Fall ist es vorteilhaft die orthopädietechnische Einrichtung so zu steuern, dass während der gesamten Schwungphase eine ausreichende Bodenfreiheit erreicht wird. Bei einem künstlichen Bein kann dies durch eine Knieflexion, eine Dorsalextension im Knöchelgelenk und/oder ggf. eine Hüftflexion erreicht werden. Bei einer für das geradeaus Gehen typischen Steuerung würde zum Beispiel das Kniegelenk in einer solchen Situationen nach dem Einbeugen zu früh strecken und der Fuß am Untergrund hängen bleiben. In einer Weiterbildung wird daher eine Rotation, insbesondere eine länger anhaltende Rotation, erkannt und daraufhin die Ansteuerung von Hüfte, Knie und/oder Fuß angepasst, insbesondere eine Hüftbeugung, eine Kniebeugung und/oder eine Dorsalflexion im Fuß nach einer Schwungphasenbeugeeinleitung gegenüber dem geradeaus Gehen unterstützt und/oder länger gehalten. Alternativ oder ergänzend werden die Bewegungen von einem Gelenk oder mehreren Gelenken verlangsamt um den Bewegungsablauf an die länger anhaltende Rotation anzupassen und ein frühzeitiges Strecken zu verhindern. Wird eine Verlangsamung und/oder ein Ende der Rotationsbewegung erkannt, werden die Widerstände und/oder die Antriebe in Hüfte, Knie und oder Fuß so angesteuert, dass das Kniegelenk in Streckung und der Fuß in eine für den Initialkontakt vorteilhafte Position gebracht werden, zum Beispiel durch das Einleiten einer Streckbewegung oder das Reduzieren eines Extensionswiderstands im Knie.

In einer Ausführungsform wird bei einer Rotation um die Längsachse der orthopädietechnischen Einrichtung und/oder einer kontralateralen Gliedmaße und/oder des Körpers ein Sondermodus verlassen. Alternativ oder ergänzend wird das Umschalten in einen Sondermodus bei einer solchen Rotation unterbunden oder abgebrochen. Orthopädietechnische Einrichtungen weisen oft mehrere Betriebsmodi auf. Neben einem Basismodus, sind ein oder mehrere Sondermodi für spezielle Bewegungsabläufe oder Aktivitäten vorhanden. Sondermodi können zyklische Bewegungsabläufe abdecken, die vom ebenen Gehen abweichen, wie das Treppensteigen, das Bergaufgehen oder das Laufen. Sondermodi können auch quasistatische Situationen abdecken, wie das Sitzen oder das gebeugte Stehen. Nicht zuletzt können Sondermodi spezielle zyklische oder nichtzyklische Aktivitäten abdecken, wie das Fahrradfahren, das Tischtennis-Spielen, das Skifahren oder einen Standby-Modus. In einem Sondermodus werden die Widerstände und Aktuatoren der orthopädietechnischen Einrichtungen anders angesteuert als im Basismodus oder beim ebenen Gehen. Für das Treppensteigen wird zum Beispiel mit der Entlastung der versorgten Seite eine aktive Flexionsbewegung mit hohem Bewegungsausmaß im Kniegelenk für das nach oben Setzen des Fußes sowie eine Streckbewegung in der darauffolgenden Standphase für das Anheben des Körpers unterstützt. In einem Fahrradmodus werden die Widerstände im Kniegelenk auf ein Minimum reduziert oder eine Streckbewegung beim nach unten Treten aktiv unterstützt. Das Umschalten in einen Sondermodus kann autonom durch die orthopädietechnische Einrichtung auf Grundlage von Sensorwerten erfolgen. Zum Beispiel kann aufgrund der erfassten Trajektorie des Fußes in der Sagittalebene in einer Schwungphase darauf geschlossen werden, dass es sich um ein Treppensteigen handelt und in einen Treppensteig-Modus geschaltet werden. Es kann auch die zyklische Pedalierbewegung beim Radfahren erkannt und in einen Radfahrmodus geschaltet werden. Das Zurückschalten in einen Basismodus kann ebenfalls autonom auf Grundlage von Sensorwerten erfolgen. Alternativ oder ergänzend kann über Bewegungsmuster, wie das mehrmalige Wippen auf dem Vorfuß, Bedienelemente und/oder externe Geräte wie Smartphones oder Tablets, die mit der orthopädietechnischen Einrichtung kommunizieren, in einen und von einem Sondermodus zurück gewechselt werden. Auch die Kombination von autonomer und nicht-autonomer Umschaltung ist möglich. Die Steuerung in einem Sondermodus ist vorteilhaft für eine spezifische Bewegung und/oder eine spezifische Aktivität. In anderen Bewegungen und/oder Situation kann diese Steuerung unvorteilhaft oder unsicher sein. Für viele Bewegungsabläufe und Situationen ist eine Rotation um die Längsachse, insbesondere eine schnelle Rotation oder eine Rotation mit hohem Bewegungsausmaß, untypisch und kann als Indikator dafür herangezogen werden, dass vom ursprünglichen Bewegungsablauf in einen anderen gewechselt werden und damit die Steuerung oder das zugrundeliegende Steuerungsgesetz angepasst werden soll. Über die Rotation um die Längsachse kann daher eine Änderung der Situation und/oder des Bewegungsmodus erkannt werden und daraufhin ein Sondermodus verlassen werden. Zum Beispiel kann beim Treppensteigen eine Rotation um die Längsachse der kontralateralen Seite erkannt werden und daraufhin der Treppensteig-Modus verlassen oder nicht aktiviert werden. Dadurch kann verhindert werden, dass in einer solchen Situation versehentlich eine Kniebeugung in der Schwungphase der versorgten Seite eingeleitet wird, insbesondere dann, wenn sich die nutzende Person umdreht und die Treppen hinunter steigen möchte. Wurde bereits eine Beugebewegung im Kniegelenk eingeleitet, bevor die Rotation erkannt wird, kann im Zuge des Verlassens des Sondermodus für das Treppensteigen aufgrund der Rotation das Kniegelenk gestreckt werden, um eine Lastübernahme auf die versorgte Seite zu ermöglichen.

Um auch bei Sondersituationen wie dem Skifahren eine angepasste Steuerung bereitzustellen, wird in einer Weiterbildung eine Extensionssperre aufgehoben, insbesondere wenn erkannt wird, dass der Tal-Ski zum Berg-Ski wird. Dies erfolgt beispielsweise durch die Detektion einer Drehung um die Längsachse der versorgten und/oder kontralateralen Gliedmaße bei einem eingebeugten Knie und/oder einer Entlastung der auf einen Unterschenkel wirkenden Axialkräfte. Wird ein Schwung erkannt, kann eine Extensionssperre des Tal-Skis aufgehoben werden, zum Beispiel wenn ein Schwellenwert einer Rotation erkannt wird.

In einer Ausgestaltung des Verfahrens wird die Orientierung bzw. die Orientierungsänderung um die Längsachse der Längserstreckung in proximal-distal Richtung der orthopädietechnischen Einrichtung und oder einer kontralateralen Gliedmaße relativ zu dem Rumpf der nutzenden Person der orthopädietechnischen Einrichtung, einer ortsfesten Komponente der orthopädietechnischen Einrichtung und/oder zu einer externen Bezugsorientierung erfasst, beispielsweise relativ zu der Schwerkraftorientierung. So kann beispielsweise bei einer Verdrehbarkeit eines Knöchelgelenkes um die Längsachse im Rahmen einer Innenrotation oder Außenrotation eine Rotation des Oberschenkels um die Längsachse verstärkt werden, wobei dies sowohl in der Schwungphase als auch in der Standphase möglich ist. Zwischen einem Prothesenkniegelenk und einem Oberschenkelteil oder Oberschenkelschaft kann ein Torsionsadapter angeordnet sein, über den eine Bei einem passiven Prothesenfuß mit einem verstellbaren Rotationswiderstand um die Längsachse kann bei Erkennen einer Rotationsbewegung in der Standphase eine Erhöhung des möglichen Bewegungsumfanges in Gestalt des Verschwenkwinkels erfolgen, um die Beweglichkeit zu erhöhen. Damit kann die entsprechende Rotation erleichtert oder überhaupt erst ermöglicht werden. Eine Veränderung des Rotationswiderstandes ist insbesondere dann sinnvoll, wenn eine Gesamtrotation der orthopädietechnischen Einrichtung erkannt wird, also wenn das Drehen auf dem Fuß der versorgten Seite erkannt wird, um dann eine erhöhte Flexibilität innerhalb der orthopädietechnischen Einrichtung zu erreichen.

In einer Ausgestaltung wird der Aktuator in Abhängigkeit von der Dauer, dem Ausmaß, der Geschwindigkeit und/oder des Geschwindigkeitsprofils der Orientierungsänderung und/oder einer Bewegung aktiviert oder deaktiviert oder wird ein Sollwert für den Aktuator moduliert. Insbesondere wird dabei die Drehung um eine Längserstreckung in proximal-distal Richtung berücksichtigt, gegebenenfalls in Verbindung mit weiteren Bewegungen oder Messgrößen, die über die Sensoren erfasst werden. Bevorzugt wird die Veränderung der Widerstände bzw. die Aktivierung des Aktuators oder die Modulierung in Kombination mit einer Vielzahl von weiteren Sensorwerten durchgeführt, um eine höhere Genauigkeit bei der Steuerung zu erzielen. Beispielsweise können der maximale Schwungphasenflexionswinkel verändert und das Niveau des Extensionswiderstandes in der Schwungphase verändert werden. Bei einer aktiven Verstellung der orthopädietechnischen Einrichtung über einen Antrieb werden beispielsweise Momente, Winkel, Positionen, Impfungen, Steifigkeiten, Geschwindigkeiten, Admittanzen oder Impedanzen als Regelungsgröße herangezogen.

In einer Ausgestaltung ist der Sensor als eine IMU ausgebildet und an der versorgten Gliedmaße, der kontralateralen Gliedmaße und/oder an dem Rumpf des Patienten angebracht. Über die IMU wird eine relative Rotation der Gliedmaße oder der Gliedmaßen zu dem Rumpf erfasst, und auf der Grundlage der erfassten Rotation wird der Widerstand entsprechend verändert oder eine Bewegung unterstützt oder eingeleitet.

Eine Weiterbildung sieht vor, dass eine Bewegungsbahn der orthopädietechnischen Einrichtung und/oder der kontralateralen Gliedmaße erfasst und der Aktivierung oder Deaktivierung des Aktuators oder der Modulierung eines Sollwertes des Aktuators zugrunde gelegt wird. Insbesondere werden die Bewegungsbahnen über eine oder mehrere IMUs oder einer Umgebungssensorik ermittelt, um eine Rotation um eine entsprechende Längsachse zu erkennen. Bei einer Drehbewegung ebenso wie einer Richtungsänderung weist auch die Bewegungsbahn oder Trajektorie der jeweiligen Komponente oder Gliedmaße, insbesondere in der Transversalebene, eine gekrümmte Form auf, sodass aus der Bewegungsbahn oder Trajektorie und deren Merkmalen wie Topologie, Form, Länge, Tangentenorientierung und/oder deren zeitlichen Änderungen und/oder Krümmung auf eine vorhandene Rotation geschlossen werden kann.

Die orthopädietechnische Einrichtung der unteren Extremität mit einem Oberteil und einem Unterteil, die gelenkig um zumindest eine Schwenkachse miteinander und Ausbildung eines Gelenkes aneinander gelagert sind und mit zumindest einem Aktuator, der mit einer Steuerungseinrichtung gekoppelt ist, die auf der Grundlage von Sensordaten zumindest eines mit der Steuerungseinrichtung gekoppelten Sensors den Aktuator aktiviert oder deaktiviert, um einen Verschwenkungswiderstand oder eine Relativbewegung des Oberteils zu dem Unterteil zu beeinflussen, sieht vor, dass der zumindest eine Sensor ausgebildet und eingerichtet ist, Sensordaten über eine Orientierung und/oder eine Veränderung der Orientierung der orthopädietechnischen Einrichtung um die Längsachse der Längserstreckung in proximal-distal Richtung der orthopädietechnischen Einrichtung und/oder einer kontralateralen Gliedmaße zu erfassen und dass die Steuerungseinrichtung eingerichtet ist, auf der Grundlage der Orientierung oder Orientierungsänderung um die Längsachse den Aktuator zu aktivieren, zu deaktivieren oder einen Sollwert für den Aktuator zu modulieren. Über den Aktuator wird das Oberteil relativ zu dem Unterteil bewegt, eine Bewegung zwischen dem Oberteil und dem Unterteil gesperrt, einer solchen Bewegung ein Widerstand entgegengebracht oder eine solche Bewegung moduliert. Dies erfolgt beispielsweise durch das Einbringen von Energie in das System. Die Veränderung der Widerstände bzw. das Antreiben erfolgt in Übereinstimmung mit der detektierten Rotationsbewegung, wobei die Steuerung nicht nur wiederholte Gangzyklen betrifft, sondern insbesondere auch Bewegungen, bei denen die orthopädietechnische Einrichtung ohne Belastung unter dem Körper neu positioniert wird. Ein Beispiel hierfür ist das Bewegen des Beines aus einer gebeugten Stellung in Kombination mit einer Drehung um die Längsachse, beispielsweise aus einer stehenden Stellung oder einer sitzenden Stellung. Bei einer solchen Bewegung ist es von Vorteil, die Kniebeugung oder Flexion einzuschränken, die Extensionsbewegung nur geringfügig zu dämpfen oder sogar zu unterstützen und gegebenenfalls eine Hüftflexion zu unterstützen, wenn aufgrund der detektierten Rotation und der jeweiligen Stellung von Oberteil und Unterteil zueinander erkannt wird, dass das Kniegelenk vor dem Aufsetzen des Fußes gestreckt oder hinreichend gestreckt sein soll. Auch für bestimmte Sportarten mit häufigen Richtungswechseln ist eine solche Steuerung vorteilhaft.

In einer Ausgestaltung ist der zumindest eine Sensor als IMU ausgebildet und an dem Oberteil oder dem Unterteil der versorgten oder der unversorgten kontralateralen Gliedmaße oder dem Rumpf der nutzenden Person befestigt und mit der Steuerungseinrichtung gekoppelt. Mit der IMU oder mit mehreren IMUs ist es möglich, Informationen hinsichtlich der Orientierung nicht nur hinsichtlich der Rotation um die Längsachse, sondern auch um andere Achsen sowie hinsichtlich der Bewegungen in unterschiedlichen Ebenen zu erlangen. Darüber hinaus ist es möglich, Positionen, Orientierungen und/oder Beschleunigungen des Oberteils und/oder des Unterteils zu erfassen oder auf der Grundlage der Sensordaten zu berechnen. Werden mehrere IMUs eingesetzt, von denen eine dem Oberteil und die andere dem Unterteil zugeordnet ist, können Winkel zwischen den Komponenten aus den ermittelten Absolutwinkeln oder Raumlagewinkeln in den jeweiligen Ebenen errechnet werden.

In einer Weiterbildung ist zumindest ein Kraftsensor, Beschleunigungssensor, Winkelsensor und/oder Momentensensor an dem Oberteil und/oder dem Unterteil angeordnet. Ein Kraftsensor kann beispielsweise zur Erfassung eines Bodenkontaktes ausgebildet sein. Ein kompressibles Element, ein verformbares oder verlagerbares Element oder auch ein elastisch gelagertes Element kann auf einen Kraftsensor oder einen als Kraftsensor dienenden Kontaktschalter einwirken, um beispielsweise zu erfassen, ob sich das jeweilige Bein in einer Standphase oder in einer Schwungphase befindet.

Eine Orientierungsänderung um die Längsachse kann sowohl eine Verdrehung einer Komponente um eine Längsachse als auch eine sich verändernde Bewegungsrichtung einer Komponente bei Bewegungen entlang einer gekrümmten Bewegungsbahn sein. Eine Orientierungsänderung um eine Längsachse kann daher auch eine in der Transversalebene gekrümmte Bewegungsbahn einer Komponente sein. Eine Orientierungsänderung kann auch eine überlagerte Bewegung aus einer Verdrehung und einer gekrümmten Bewegungsbahn sein. Die Orientierung kann die momentane Bewegungsrichtung bei Bewegung entlang einer gekrümmten Bewegungsbahn sein. Die Orientierung um eine Längsachse ist entsprechend die Bewegungsrichtung in der Transversalebene. Eine Transversalebene ist eine Ebene normal auf eine Längsachse. Eine Rotation oder Rotationsbewegung um eine Längsachse ist gleichbedeutend mit einer solchen Orientierungsänderung um die Längsachse.

Bei einer Rotation um die Längsachse der Längserstreckung in proximal-distal-Richtung der orthopädietechnischen Einrichtung oder der kontralateralen Seite oder des Körpers kann sich die Längsachse sowohl auf die momentane Längsachse beziehen, welche bei einer Verschwenkung der orthopädietechnischen Einrichtung und/oder der kontralateralen Seite in der Sagittalebene und/oder Frontalebene mit verschwenkt wird als auch auf die Längsachse in einer Referenzposition, beispielsweise im aufrechten Stand.

Wird die orthopädietechnische Einrichtung auf Basis einer Orientierung und/oder Orientierungsänderung um eine Längsachse gesteuert ist es möglich, dass ein gewisses Mindestmaß an Bewegung oder Änderung der Bewegung mit der Zeit und deren beliebige Ableitungen notwendig ist, um einen Einfluss auf die Steuerung zu haben. Dies kann über einen oder mehrere Schwellwerte und/oder über komplexere Algorithmen umgesetzt werden, bspw. über einen Mehrheitsentscheid über mehrere Werte oder über künstliche Intelligenz.

Sämtliche Steuerungsalgorithmen, welche eine Orientierung und/oder Orientierungsänderung um eine Längsachse oder daraus abgeleitete Größen als Eingangsgröße aufweisen, können auch weitere Eingangsgrößen haben, insbesondere Bewegungen in andere Richtungen, Belastungen und/oder Informationen von anderen Sensoren, welche Einfluss auf das Verhalten der orthopädietechnischen Einrichtung haben.

Nachfolgend werden Ausführungsbeispiele der Erfindung anhand der Figuren näher erläutert. Es zeigen:
Figur 1 - eine schematische Darstellung eines Prothesenbeines;
Figur 2 - eine schematische Darstellung einer KAFO;
Figur 3 - einen ersten Bewegungsablauf für das Gehen um eine Kurve;
Figur 4 - eine schematische Frontalansicht eines zweiten Zustandes;
Figur 5 - einen zweiten Bewegungsablauf aus dem Zustand der Figur 4;
Figur 6 - unterschiedliche Bewegungsabläufe;
Figuren 7 und 8 - weitere Ausführungsbeispiele von Bewegungsabläufen;
Figur 9 - eine Erfassung der Orientierung in der Transversalebene;
Figur 10 - eine Darstellung einer relativen Rotation von Rumpf und ipsilateraler Seite;
Figur 11 - eine Trajektorie einer ipsilateralen Seite;
Figuren 12 bis 14 - verschiedene Parameterverläufe;
Figur 15 - eine Darstellung eines Bewegungsablaufes bei einer Drehung auf einer Treppe;
Figur 16 - eine Darstellung einer Deaktivierung bei einer Drehung;
Figur 17 - ein Anwendungsbeispiel für eine Steuerungsanpassung;
Figur 18 - eine Darstellung von Rotationsachsen in der Frontalebene;
Figur 19 - eine Darstellung von Rotationsachsen in der Sagittalebene; sowie
Figur 20 - zwei weitere Bewegungsabläufe.

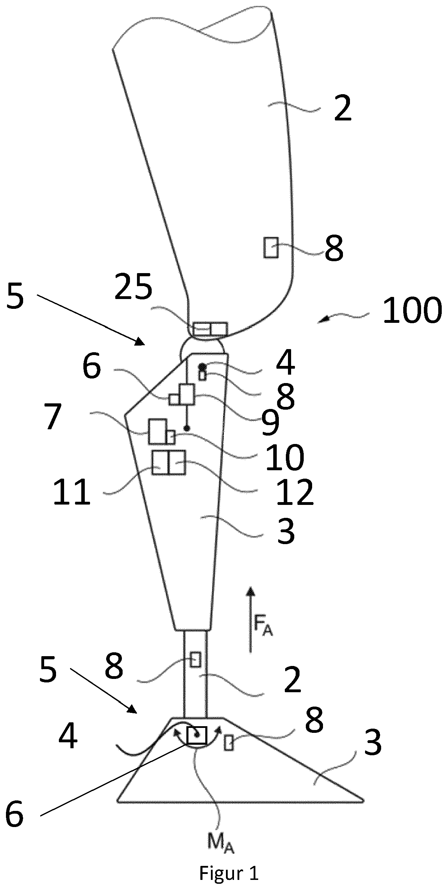

Figur 1 zeigt eine schematische Darstellung einer orthopädietechnischen Einrichtung 100 in Gestalt eines Prothesenbeines mit einem ersten Oberteil 2 in Gestalt eines Oberschenkelschaftes und einem ersten Unterteil 3 in Gestalt eines Unterteils eines Prothesenkniegelenkes 5. Das Oberteil 2 ist schwenkbar zu dem Prothesenunterteil 3 um eine Schwenkachse 4 gelagert. An dem Oberteil 2 sind
Befestigungseinrichtungen 25 zur Festlegung des Oberschenkelschaftes an dem Prothesenkniegelenk 5 angeordnet oder ausgebildet. Die Befestigungseinrichtungen 25 sind beispielsweise ein Pyramidenadapter mit einer entsprechenden Aufnahme. Das erste Unterteil 3 in Gestalt eines Unterschenkelteils weist ein Unterschenkelrohr an seinem distalen Ende auf, das wiederum als zweites Oberteil 2 für eine gelenkige Verbindung mit einem Prothesenfuß als zweites Unterteil 3 dient. Der Prothesenfuß 3 ist schwenkbar um die Knöchelgelenksachse als zweite Schwenkachse 4 gelagert. Die schwenkbare Verbindung von Unterschenkelrohr und Prothesenfuß bildet das Knöchelgelenk 5 aus. Somit weist die orthopädietechnische Einrichtung 100 zwei Oberteile 2 und zwei Unterteile 3 auf, wobei das Unterschenkelteil als Verbindung zwischen den beiden Schwenkachsen 4 einteilig oder mehrteilig ausgebildet sein kann und je nach Betrachtungsweise einmal das Unterteil und einmal das Oberteil ist.

Die gelenkige Verbindung von Oberteil 2 und Unterteil 3 um die jeweilige Schwenkachse 4 miteinander bildet das jeweilige Gelenk 5 aus. In dem dargestellten Ausführungsbeispiel ist zwischen dem Oberteil 2 und dem Unterteil 3 des Kniegelenkes eine Widerstandseinrichtung 9 in Gestalt eines verstellbaren Dämpfers angeordnet. Die Widerstandseinrichtung 9 stützt sich mit einer proximalen Anschlusseinrichtung an dem Oberteil 2 und mit einer distalen Anschlusseinrichtung an dem Unterteil 3 ab. Die Widerstandseinrichtung 9 ist in dem Ausführungsbeispiel als passives Bauteil ausgebildet und beeinflusst eine Verschwenkbewegung des Oberteils 2 relativ zu dem Unterteil 3 um die Schwenkachse 4 sowohl in Flexionsrichtung als auch in Extensionsrichtung durch Umwandlung kinetischer Energie in Wärmeenergie. Der Widerstandseinrichtung 9 ist ein Aktuator 6 zur Verstellung des jeweiligen Widerstandes zugeordnet. Der Aktuator 6 wirkt auf die Widerstandseinrichtung 9 entsprechend dem Wirkprinzip ein. Ist die Widerstandseinrichtung 9 beispielsweise als eine pneumatische oder hydraulische Dämpfereinrichtung ausgebildet, verändert der Aktuator 6 den Strömungsquerschnitt der Leitung von einer Extensionskammer in die Flexionskammer und zurück, um so den jeweiligen Strömungsquerschnitt eines Überströmkanals zu vergrößern und zu verkleinern. Dadurch wird der Strömungswiderstand verkleinert oder vergrößert. Alternativ oder ergänzend zu einer Veränderung des Strömungsquerschnittes kann der Aktuator 6 oder ein Aktuator 6 als verstellbarer Magnet ausgebildet sein, z.B. als Elektromagnet, der auf eine magnetorheologische Flüssigkeit einwirkt. Durch Veränderung des Magnetfeldes verändert sich die Viskosität der magnetorheologischen Flüssigkeit, sodass über die Veränderung der Viskosität der Verschwenkungswiderstand verändert wird. Die Widerstandseinrichtung 9 kann auch als ein Elektromotor ausgebildet sein, der im Generatorbetrieb betrieben werden kann, bei der Flexionswiderstand und/oder Extensionswiderstandes durch eine entsprechende Generatorregelung verändert wird. In diesem Fall ist in der Regel der Generator der Aktuator. Ist eine rein mechanische Bremse, beispielsweise ein Reibungsbremse, vorgesehen, bei der Bremsbeläge gegen eine sich bewegende Komponente gepresst werden, ist der Aktuator der Motor oder Antrieb, mit dem die Bremsbeläge gegen die Komponente gedrückt werden.

Alternativ oder ergänzend zu einer rein passiven Ausgestaltung der Widerstandseinrichtung kann der Aktuator 6 auch als aktives Element, z.B. als Elektromotor ausgebildet sein, um eine Relativbewegung des Oberteils 2 zu dem Unterteil 3 nicht nur beeinflussen, sondern auch aktiv hervorzurufen. Alternativ zu einer Ausgestaltung als Elektromotor kann der Aktuator 6 auch andere Antriebseinrichtungen oder -prinzipien zur Freisetzung gespeicherter Energie nutzen.

Der Aktuator 6 wird über eine Steuerungseinrichtung 7 aktiviert, deaktiviert oder moduliert. Je nach Signal von der Steuerungseinrichtung wird die Flexion und/oder Extension beeinflusst und gegebenenfalls blockiert. Durch die Steuerungseinrichtung 7 mit dem entsprechenden Signal wird das Bewegungsverhalten des jeweiligen Gelenkes 5 während des Gehens, Stehens oder einer anderen Nutzung eingestellt. Der Steuerungseinrichtung 7 sind Sensoren 8 zugeordnet, die auf der gesamten orthopädietechnischen Einrichtung 100 angeordnet sind. Die Sensoren 8 liefern entsprechende Daten drahtlos oder über Kabelverbindungen an die Steuerungseinrichtung 7. Die Daten der Sensoren 8 können vorverarbeitet und/oder in der Steuerungseinrichtung 7 selbst verarbeitet werden. In der Steuerungseinrichtung 7 sind Prozessoren, Speicher und alle anderen notwendigen Komponenten vorhanden oder mit ihr gekoppelt, um die Sensordaten auszuwerten und auf der Basis dieser Auswertung eine entsprechende Aktivierung, die Deaktivierung oder Modellierung des Aktuators und damit der Widerstandseinrichtung 9 vorzunehmen.

Die Steuerungseinrichtung 7 weist insbesondere auch eine Speichereinrichtung 10 auf und kann mit einem Sender 11 und einem Empfänger 12 gekoppelt sein, um Sensordaten, Programme, Zugriffsrechte, Einstellungen, Änderungen von Einstellungen, Updates oder anderes auf externe Komponenten oder auf Komponenten innerhalb der orthopädietechnischen Einrichtung zu übertragen. Die Sensoren 8 erfassen während der Nutzung der orthopädietechnischen Einrichtung sämtliche relevanten Parameter, beispielsweise Kräfte, Momente, Beschleunigungen, Temperaturen, Zeiten, Orientierungen im Raum, Verformungen, Bewegungszeiträume, Nutzungszeiträume, Distanzen, relative Bewegung, Interaktionen mit der Umgebung, Spannungen, Ströme, Biosignale, elektromagnetische Strahlung und dergleichen. Insbesondere sind die Sensoren 8 oder Sensoreinrichtungen als Komponenten ausgebildet, die eine Winkelstellung der Komponenten zueinander und/oder eine Raumlage oder eine Orientierung im Raum erkennen. Zusätzlich sind die Sensoren 8 zur Erfassung von Axialkräften FA und Momenten MA ausgebildet. Die Kräfte und Momente werden überall dort ermittelt, wo die Erfassung sinnvoll und notwendig ist, auch wenn diese Kräfte und Momente nur im Zusammenhang mit dem Knöchelgelenk eingezeichnet sind. Nicht alle Sensoren 8 können alle Parameter erfassen, die Anordnung und Ausgestaltung der Sensoren richtet sich nach den jeweils zu erfassenden Parametern.

Aus Sensorwerten können auch abgeleitete Größen errechnet werden. Beispielsweise können aus Kraft- und/oder Momentenkomponenten Hebelarme zu gewissen Punkten und/oder Kraftangriffspunkte berechnet werden, Sensorwerte zu Kenngrößen fusioniert werden, wie beispielsweise in IMUs (Inertial Measurement Units), aus Verformungen auf Kräfte rückgerechnet werden und/oder aus mehreren Abständen durch Triangulation auf eine Position zurück gerechnet werden. Derartige errechnete Größen sind in den beschriebenen Ausführungen mit eingeschlossen und können für die Steuerung der orthopädietechnischen Einrichtung herangezogen werden, insbesondere zur Steuerung von Bewegungsabläufen mit einer Verschwenkung in der Frontalebene.

In dem Ausführungsbeispiel ist an dem Knöchelgelenk als Aktuator 6 ein Elektromotor angeordnet, über den je nach Bedarf über den Generatorbetrieb eine Widerstandseinrichtung und im Motorbetrieb eine Unterstützung oder eine aktive Verlagerung des Prothesenfußes relativ zu dem Unterschenkelteil um die Schwenkachse 4 bereitgestellt wird.

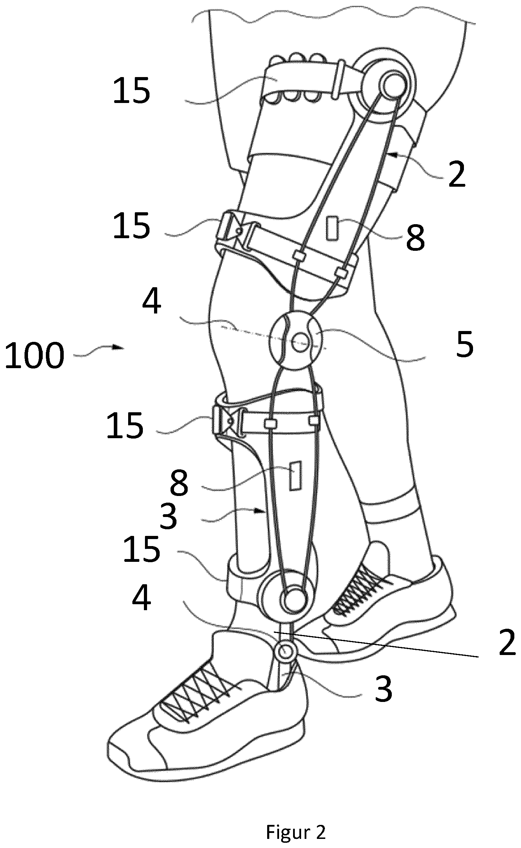

In der Figur 2 ist eine orthopädietechnische Einrichtung 100 als eine Orthese der unteren Extremität in einem angelegten Zustand gezeigt. Es handelt sich hierbei um eine KAFO (Knee Ankle Foot Orthosis), bei der ein erstes Oberteil 2 in Gestalt einer Oberschenkelschiene über Befestigungseinrichtungen 15 in Gestalt von Gurten an einem Oberschenkel festgelegt ist. Ein erstes Unterteil 3 in Gestalt einer Unterschenkelschiene ist ebenfalls über Befestigungseinrichtungen 15 an einem Unterschenkel einer nutzenden Person angeordnet. Die Oberschenkelschiene und die Unterschenkelschiene sind um eine Schwenkachse 4 unter Ausbildung eines Orthesenkniegelenkes 5 schwenkbar aneinander befestigt. An oder in dem Orthesenkniegelenk 5 sind die in der Figur 1 erläuterten Komponenten und technischen Einrichtungen wie Aktuator, Widerstandseinrichtung, Steuerungseinrichtung, Schnittstellen und dergleichen angeordnet. Die Sensoren 8 sind schematisch dargestellt. Die zweite Schwenkachse 4 im Bereich des natürlichen Knöchelgelenkes verbindet die Unterschenkelschiene als zweites Oberteil 2 mit einem Fußteil als zweites Unterteil 3. Die Einrichtung zur Beeinflussung des Prothesenknöchelgelenkes hinsichtlich des Widerstandes in Richtung der Plantarflexion oder der Dorsalflexion ist im Bereich des Orthesenknöchelgelenkes untergebracht. Auch hier können passive Widerstandseinrichtungen und/oder aktive Antriebe oder Aktuatoren vorgesehen sein.

Sowohl bei der Ausgestaltung als Prothese als auch bei der Ausgestaltung als Orthese können bei mehreren Gelenken 5 und entsprechenden Widerstandseinrichtungen die Aktuatoren 6 zur Beeinflussung der Verschwenkbewegung um die jeweilige Schwenkachse 4 von einer gemeinsamen Steuerungseinrichtung 7 gesteuert werden. Ebenso ist es möglich, dass mehrere Steuerungseinrichtungen 7 ausgebildet oder angeordnet sind, um die orthopädietechnische Einrichtung 100 entsprechend zu steuern.

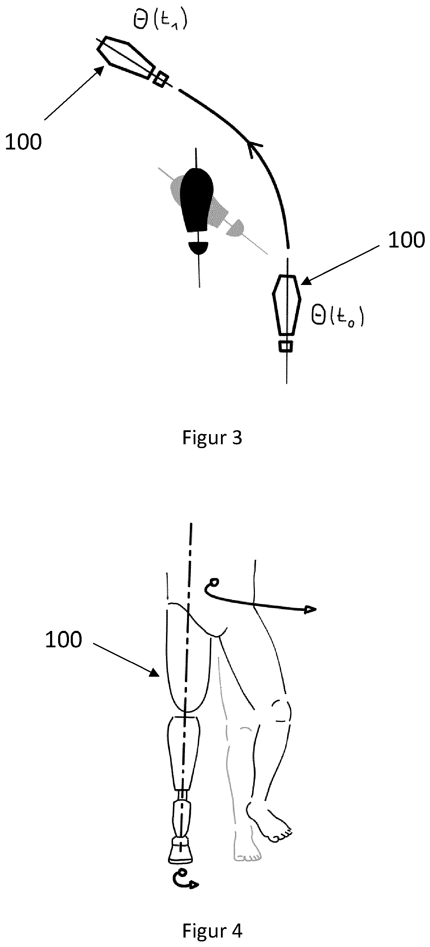

In der Figur 3 ist schematisch ein erster Bewegungsablauf einer Person mit einer orthopädietechnischen Einrichtung 100 in Gestalt eines Prothesenbeins, ähnlich dem in der Figur 1, dargestellt. Die nutzende Person der orthopädietechnischen Einrichtung 100 steht im Wesentlichen gerade und aufrecht. In der dargestellten Stellung ist das von der nutzenden Person aus gesehen linke Bein das unversorgte Bein. Eine Vorwärtsbewegung wäre eine Bewegung gerade nach vorne. Möchte nun die nutzende Person eine Bewegungsänderung durchführen oder aus einer stehenden Position sich nach links wenden, wird zu einem Zeitpunkt t=0 zunächst das rechte, versorgte Bein mit der orthopädietechnischen Einrichtung 100 angehoben, in einem Bogen nach vorne und links bewegt und zu einem Zeitpunkt t₁ abgesetzt.

Nach dem Aufsetzen des Prothesenfußes der orthopädietechnischen Einrichtung 100 verlagert die nutzende Person das Gewicht auf die versorgte Seite und zieht ihren linken, unversorgten Fuß neben den Prothesenfuß. Dabei oder während der Schwenkbewegung der versorgten Seite um die Längsachse des Standbeines kann der linke Fuß des Standbeines ebenfalls verschenkt werden. Dazu wird in der Regel der Vorderfuß belastet und eine Drehung um die Längsachse der Längserstreckung des Standbeines auf dem Ballen ausgeführt. Dies ist durch die übereinander dargestellten Fußabdrücke angedeutet. Die Rotation um die Längsachse der Längserstreckung des Standbeines oder der kontralateralen Seite wird beispielsweise über eine IMU erfasst, die an der orthopädietechnischen Einrichtung 100 angeordnet ist. Sofern eine weitere IMU an der kontralateralen Gliedmaße befestigt ist, kann auch eine relative Verdrehung der versorgten Seite zu der unversorgten Seite erfasst werden, sodass berücksichtigt wird, dass eine Drehung um das Standbein mit ausgeführt wird. Wird eine Rotationsbewegung um eine Längsachse der Längserstreckung detektiert, kann beispielsweise der Flexionswiderstand verringert werden, um eine vergrößerte Knieflexion zu ermöglichen. Dies führt dazu, dass zwischen dem Anheben der versorgten Seite und dem erneuten Aufsetzen zu dem Zeitpunkt t₁ ein längerer Weg zurückgelegt werden muss, sodass sich ein verzögerter oder längerer Bewegungsablauf ergibt. Nach dem Anheben des Prothesenfußes wird z.B. eine Flexionsbewegung innerhalb des Knies erleichtert oder eingeleitet, indem der Flexionswiderstand verringert oder eine aktive Flexionsunterstützung durch den nicht dargestellten Aktuator initiiert wird. Die Erkennung des Anhebens erfolgt beispielsweise durch eine Überwachung des Axialkraftverlaufes innerhalb des Prothesenunterteils oder des Prothesenfußes in Verbindung mit der Überwachung einer Bewegung und/oder Stellung der orthopädietechnischen Einrichtung 100. Über die Rotationsgeschwindigkeit um die Längsachse der Längserstreckung der unversorgten Seite kann abgeschätzt werden, wie groß der Zeitraum zwischen dem Anheben (t₀) und dem Aufsetzen (t₁) sein wird, sodass eine Erhöhung oder Verringerung des Flexionswiderstandes und/oder eine Verringerung eines Extensionswiderstandes oder eine Aktivierung eines Antriebes erfolgen kann. Dazu wird über die Steuerungseinrichtung ein entsprechendes Signal erzeugt und an den Aktuator übermittelt. In einer Ausführungsform des Verfahrens wird der Flexionswiderstand in dem künstlichen Kniegelenk erhöht und/oder eine Extension in dem künstlichen Kniegelenk bewirkt, sobald eine Axialbelastung oder ein Aufsetzen des Fußes der versorgten Seite detektiert wird. Dadurch wird eine Abschätzung der Bewegungsdauer entbehrlich, da immer erkannt wird, wann eine Rotationsbewegung um das Standbein beendet ist.

Bei einer Bewegung des Prothesenfußes ist in einer Ausgestaltung des Verfahrens vorgesehen, eine Plantarflexion zu unterbinden und auch eine Dorsalflexion zu bewirken, damit der Prothesenfuß oder eine Fußplatte einer Orthese vollflächig oder mit einer geraden Sohle im Wesentlichen parallel zum Untergrund aufgesetzt werden kann. Alternativ kann die Bewegung von Stellung t₀ zu der Stellung t bei einem aktiven Fuß mit einer Plantarflexion verbunden sein, sodass zunächst eine Fußspitze aufsetzt und bei einer zunehmenden Belastung eine Dorsalflexion stattfindet. Es ist auch möglich dass der Fuß während der Bewegung in einer leicht nach unten zeigender Position gehalten wird oder in diese gebracht wird.

In der Figur 4 ist eine alternative Position dargestellt, bei der die versorgte Seite mit der orthopädietechnischen Einrichtung 100 das rechte Bein darstellt. Das linke Bein als kontralaterale Seite ist angehoben, sodass die versorgte Seite das Standbein darstellt. Wird nun die unversorgte, linke Seite dergestalt bewegt, dass der Fuß nach vorne und links gesetzt und verdreht wird, bedeutet dies, dass um die Längserstreckung des Standbeines herum eine Rotationsbewegung stattfinden muss. Um diese Rotationsbewegung zu erleichtern, wird ein Widerstand in einem Knöchelgelenk oder einem künstlichen Kniegelenk um eine Längsachse verringert und eine entsprechende Drehung zugelassen oder erleichtert. Dadurch wird bei einem aufgesetzten Prothesenfuß eine Drehung des Oberschenkels und damit des gesamten Rumpfes erleichtert. Wird die Rotation um die Längsachse detektiert oder eine Rotation der unversorgten Seite erfasst, wird in einer Ausgestaltung zur Vergrößerung der Stabilität der Flexionswiderstand innerhalb des künstlichen Kniegelenkes angepasst, beispielsweise vergrößert, sodass ein ungewolltes Einsinken unterbleibt. Zur Erleichterung der Drehbewegung erfolgt in einer Ausgestaltung eine Unterstützung einer Extensionsbewegung oder zumindest eine Verringerung eines Extensionswiderstandes, sodass die versorgte Seite leichter in eine maximale Streckung gebracht werden kann.

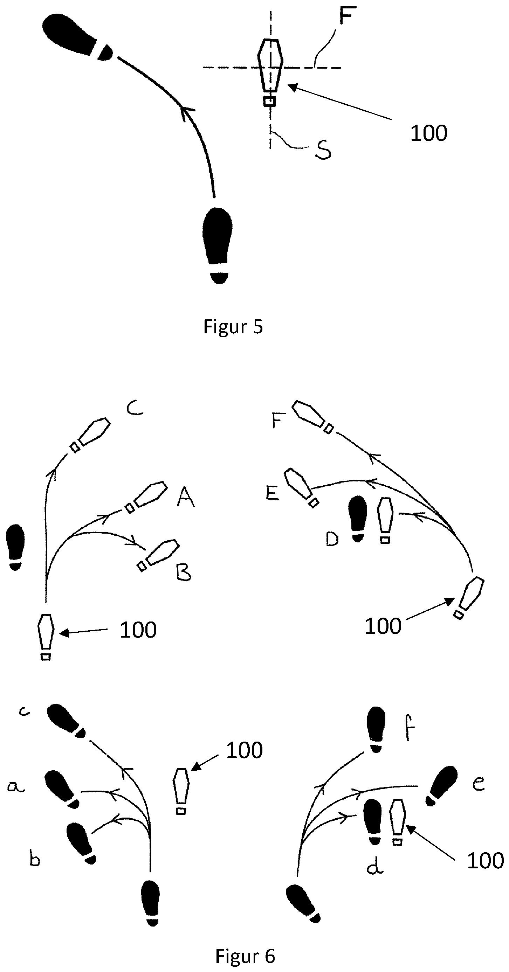

In der Figur 5 ist das Bewegungsmuster einer Kurvenbewegung mit einem aufgesetzten Prothesenfuß schematisch dargestellt. Der Prothesenfuß der orthopädietechnischen Einrichtung 100 befindet sich in einer Stellung, die in Gehrichtung nach vorne zeigt, die Längserstreckung des Prothesenfußes liegt innerhalb der Sagittalebene S, die Frontalebene F verläuft senkrecht dazu. Wird das Anheben des linken, unversorgten Fußes detektiert oder kann darauf geschlossen werden, beispielsweise durch einen Anstieg der Axialbelastung in der orthopädietechnischen Einrichtung 100, werden die entsprechenden Steuerungssignale an den Aktuator gesendet. Wird gleichzeitig eine Rotation der unversorgten Seite und/oder eine Rotation um die Längsachse der Längserstreckung der versorgten Seite über die Sensoren detektiert, kann die Beweglichkeit um die Längsachse in den künstlichen Gelenken vergrößert werden, beispielsweise durch die Verringerung entsprechender Widerstände. In einer Ausgestaltung wird der Flexionswiderstand um die Knieachse gegen eine Dorsalflexion im Knöchelgelenk vergrößert, um ein ungewolltes Einbeugen in den jeweiligen Gelenken zu vermeiden und den Richtungswechsel zu erleichtern. Bei der dargestellten Außenrotation mit der kontralateralen Seite ist es günstig, wenn sich die nutzende Person von der versorgten Seite abdrücken kann, um die Impulsänderung zu bewerkstelligen. Zudem ist auf der versorgten Seite eine geringe Abrollbewegung nach vorne notwendig. Durch die Anhebung der Widerstände wird dies bewerkstelligt. Wird nach dem Aufsetzen der unversorgten Seite die orthopädietechnische Einrichtung 100 nachgeführt und wieder parallel zu dem vorderen, linken Fuß bewegt, kann für diese Bewegung, die länger als ein normaler Schritt beim Gehen geradeaus dauern kann und in der andere Kräfte und Momente auf die Hilfseinrichtung wirken können als beim geradeaus Gehen, eine Modifikation der Extensionswiderstände und Flexionswiderstände bzw. der aktiven Verschwenkung in Extensionsrichtung und Flexionsrichtung erfolgen.

In der Figur 6 sind unterschiedliche Bewegungsmuster für eine Kombination aus Rotationsbewegungen, Vorwärtsbewegungen und seitlichen Bewegungen innerhalb Transversalebene dargestellt. Es handelt sich damit um krummlinige Bewegungen oder Kurvenbewegungen in der Transversalebene. In der oberen Bewegungsreihe sind die Bewegungen von der versorgten Seite ausgeführt und mit Großbuchstaben gekennzeichnet, in der unteren Reihe ist die versorgte Seite das Standbein und die unversorgte Seite wird bewegt, was mit Kleinbuchstaben gekennzeichnet ist. Grundsätzlich sind die Bewegungen auch umgekehrt oder seitenverkehrt durchführbar. Bei der Bewegung A wird bei einem hinten stehenden Prothesenfuß, beispielsweise aus einer Schrittstellung, bei dem die unversorgte Seite vorne ist, die versorgte Seite sowohl nach vorne als auch seitlich bis auf die Höhe der unversorgten Seite bewegt, sodass sich eine Kurvenbewegung einstellt. Bei der Bewegung B findet eine Bewegungsumkehr der Vorwärtsbewegung statt, sodass die versorgte Seite, beispielsweise der Prothesenfuß, schräg hinter den unversorgten Fuß gestellt wird. Bei der Bewegung C wird die versorgte Seite geradlinig in der Sagittalebene bis zur Höhe des unversorgten Fußes geführt und dann schräg nach vorne abgesetzt. In allen drei Bewegungen findet eine Verschwenkung der versorgten Seite um eine Längserstreckung der Längsachse sowohl der versorgten als auch der unversorgten Seite statt.

Bei den Bewegungen D bis F ist die Ausgangsposition der versorgten Seite mit einem Prothesenfuß schräg hinter der unversorgten Seite. Die versorgte Seite wird in der Bewegung D seitlich neben den unversorgten Fuß in einer Kreisbahnbewegung geführt, bei der Bewegung E vor den unversorgten Fuß im Rahmen eines Kreuzschrittes. Bei der Bewegung F wird der versorgte Fuß übergesetzt und schräg vor den unversorgten Fuß gesetzt. In allen Bewegungen befindet sich die versorgte Seite in der Ausgangsposition aufgrund des breitbeinigen Standes in einer in der Frontalebene geneigten Stellung und führt eine Rotationsbewegung oder eine Bewegung auf einer gekrümmten Bahn aus, die von dem üblichen Bewegungsmuster des vorwärts Gehens abweicht und mit einer entsprechend angepassten Steuerung der Widerstände und/oder Antriebe begegnet werden muss.

Die gleichen oder entsprechende Bewegungen werden in der unteren Reihe mit der unversorgten Seite ausgeführt, der Prothesenfuß oder der Fußteil der versorgten Seite ist dabei die stehende Komponente. Die Bewegungen sind entsprechend ausgeführt und können in beide Richtungen ausgeführt werden, also statt einem Heranziehen ein Wegsetzen und umgekehrt. Bei Bewegungen mit der unversorgten Seite in der Schwungphase erfolgt über eine Drehung im Knöchelgelenk mit einer fixierten Position, über eine Drehung um einen Fußaufsatzpunkt oder einem COP oder in dem Hüftgelenk der versorgten Seite durch eine Drehung des gesamten Rumpfes.

Mit der Berücksichtigung von Rotationsbewegungen um die Längsachse einer Längserstreckung einer orthopädietechnischen Einrichtung, einer kontralateralen Seite oder einer relativen Drehung mehrerer Komponenten oder Gliedmaßen zueinander und gegebenenfalls des gesamten Rumpfes ist es möglich, den besonderen Bedingungen bei Bewegungen Rechnung zu tragen, die von dem Gehen in der Ebene in einer geraden Richtung abweichen. Insbesondere bei dem Gehen von Kurven wird häufig die unversorgte Seite als Standbein genutzt, sodass eine Innenrotation um die unversorgte Seite ausgeführt wird. Durch die dabei ausgeführte Drehbewegung in der Schwungphase der versorgten Seite wirken andere Kräfte und Momente auf die Gelenke der orthopädietechnischen Einrichtung als beim geradeaus Gehen, was zu der Notwendigkeit der Anpassung der Standardsteuerung führt. Durch die Drehbewegung kann beispielsweise der Unterschenkel stärker aufschwingen bzw. das Kniegelenk länger in der gebeugten Stellung bleiben, sodass die Gefahr besteht, dass sich der Fuß der versorgten Seite nicht rechtzeitig in der vorgesehenen Stellung bei einem Initialkontakt befindet. Es muss daher die Beeinflussung der Verschwenkbewegungen der Gelenke über Widerstände und Antriebe verändert werden, sodass ein rechtzeitiges Strecken des Gelenks vor Initialkontakt bewerkstelligt wird. Das Gelenk kann zum Beispiel schneller in Streckung gebracht werden oder der Flexionswiderstand vergrößert werden, um ein zu starkes oder langes Aufschwingen zu verhindern. Unter Verwendung von Informationen über die Orientierung bzw. die Änderung der Orientierung um die Längsachse der Längserstreckung der orthopädietechnischen Einrichtung, der kontralateralen Seite bzw. einer Referenzorientierung kann die Ansteuerung der Widerstände und Antriebe optimiert werden. Bei der orthopädietechnischen Einrichtung kann es sich sowohl um passive als auch um aktive Gelenke oder Gelenkssysteme handeln.

Besonders vorteilhaft ist die Veränderung der Widerstände oder Aktivierung bzw. Deaktivierung von Antrieben bei der orthopädietechnischen Einrichtung in der Schwungphase der versorgten Seite, wenn diese ohne Bodenkontakt bewegt wird. Bei Bewegungen, die eine Rotation um eine Längsachse beinhalten, die im wesentlichen proximal-distal verläuft, verhält sich die unter dem Körper befindliche orthopädietechnische Einrichtung anders als bei geradlinigen Bewegungen beispielsweise in der Sagittalebene. Bei einer Rotation handelt es sich um eine beschleunigte Bewegung, die insbesondere die Pendeldauer beeinflusst. Während bei einer geradlinigen Bewegung das gesamte Ausmaß der Gehbewegung durch die Abrollbewegung der kontralateralen Seite und die Schrittlänge beschränkt ist, können Rotationsbewegungen selbst bei hohen Drehgeschwindigkeiten wesentlich länger dauern, beispielsweise bei einer Umkehr der Gehrichtung oder bei einer einfachen oder mehrfachen Drehung um die eigene Achse.

Die Anpassung der Widerstände oder die Aktivierung und Deaktivierung von Antrieben erfolgt nicht nur bei sich wiederholenden Gangzyklen, sondern auch bei Bewegungen, bei denen die orthopädietechnische Einrichtung ohne Belastung unter dem Körper repositioniert wird. Auch bei dem Gehen auf Rampen oder Treppen mit Rotationsbewegungen erfolgt eine Anpassung der Widerstände oder Antriebe.

Auch Knöchelgelenke können bei einer Detektion von Rotationen um eine Längsachse, beispielsweise um die Längsachse des Unterschenkelteils, hinsichtlich ihres Widerstandes oder ihrer Beweglichkeit modifiziert werden. Besteht ein Rotationsfreiheitsgrad um die Längsachse des Unterschenkels oder Unterschenkelteils kann dieser vergrößert werden, wenn eine Innenrotation oder Außenrotation detektiert wird. Bei einer Außenrotation des Oberschenkels entweder absolut oder relativ zu dem Körper kann auch der Fuß nach außen rotiert werden, bei einer Innenrotation entsprechend umgekehrt. Vor allem bei einer Außenrotation auf dem kontralateralen Standbein kann dies vorteilhaft sein, wobei die Außenrotation und Innenrotation sowohl in der Schwungphase als auch in der Standphase für das Knöchelgelenk erfolgt.

In einer Ausgestaltung wird nur die relative Änderung der Orientierung zwischen zwei Zeitpunkten gemessen, beispielsweise zwischen dem Toe-Off und dem erneuten Aufsetzen des Fußes. Beträgt die Verdrehung zwischen diesen beiden Positionen mehr als ein Schwellenwert, beispielsweise mehr als 90°, wird eine Anpassung der Steuerung vorgenommen, eine absolute Bestimmung der Himmelsrichtung oder Orientierung zu einer Bezugsgröße ist dabei nicht erforderlich.

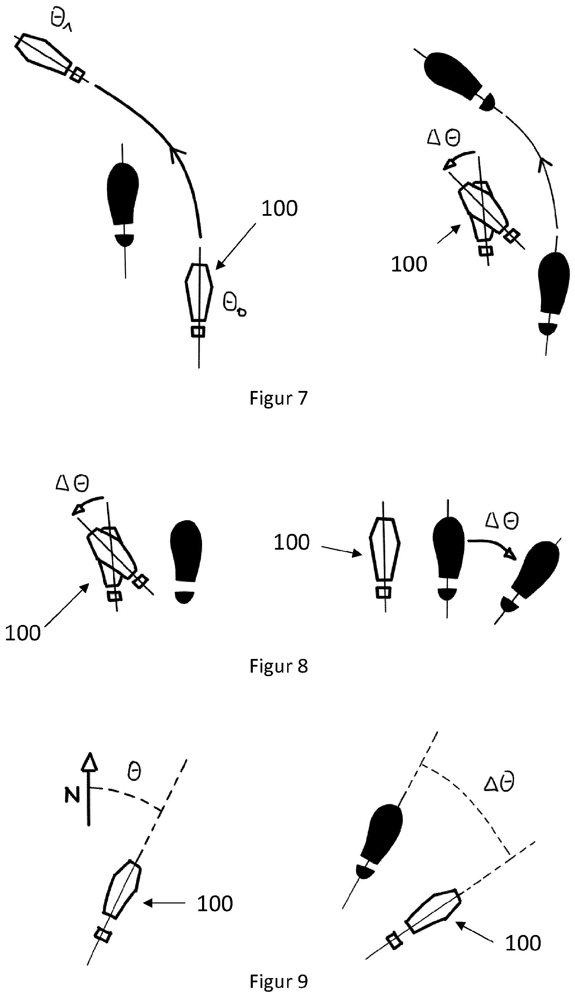

In der Figur 7 ist links eine schematische Darstellung eines weiteren Bewegungsablaufes gezeigt, bei der eine Drehung in der ipsilateralen Schwungphase der orthopädietechnischen Einrichtung 100 bzw. der Gliedmaße, an dem die orthopädietechnische Einrichtung 100 angeordnet ist, erfolgt. Zu einem ersten Zeitpunkt liegt eine erste Orientierung θ₀ vor. Beide Füße befinden sich in einer nahezu parallelen Ausrichtung in Richtung der Sagittalebene in Schrittstellung. Die versorgte Seite mit der orthopädietechnischen Einrichtung 100 wird zu einem Anfangszeitpunkt in der Orientierung θ₀ angehoben und ausgehend von der Ausgangsorientierung nach vorne und um etwa 60° entgegen dem Uhrzeigersinn um die versorgte, kontralaterale Seite herum bewegt. Die abschließende Orientierung θ₁ zu einem zweiten Zeitpunkt ist oben links in der Figur dargestellt. In der rechten Darstellung der Figur 7 ist die gleiche bzw. eine entsprechende Bewegung des unversorgten, rechten Bein dargestellt. Die orthopädietechnische Einrichtung 100 an dem versorgten Bein führt dabei eine Rotation auf dem Standbein um einen Winkel Δθ aus, während sich das kontralaterale Bein in der Schwungphase befindet. In beiden Fällen oder bei beiden Bewegungsabläufen findet ein Gehen auf einer gekrümmten Bahn oder einer Kurve statt, ebenso wird eine Orientierung bzw. Orientierungsänderung um die Längsachse der Längserstreckung der orthopädietechnischen Einrichtung bzw. der kontralateralen Seite erkannt. Bei der Bewegung in der linken Darstellung der Figur 7 wird die orthopädietechnische Einrichtung 100 um die Längsachse der Längserstreckung der Gliedmaße der kontralateralen Seite verschwenkt und damit auch noch auf einer Kreisbahn bewegt, während in der rechten Darstellung die orthopädietechnische Einrichtung 100 nur um die Längsachse der Längserstreckung gedreht wird. Die Längsachse der Längserstreckung der orthopädietechnischen Einrichtung 100 in der rechten Darstellung bleibt also stationär, in der linken Darstellung ist sie instationär, da eine Drehung um die kontralaterale Achse erfolgt.

In der Figur 8 sind ebenfalls zwei Situationen dargestellt, bei der eine Drehung um die Längsachse der Längserstreckung der orthopädietechnischen Einrichtung 100 erfolgt. In der linken Darstellung findet eine Drehung auf der ipsilateralen Seite bei einem beidbeinigen Stehen statt. Beide Füße befinden sich auf dem Boden, die linke, versorgte Seite wird, um die Längsachse der Längserstreckung verdreht, in dem dargestellten Ausführungsbeispiel um den Winkel Δθ entgegen dem Uhrzeigersinn. Es ist auch möglich, dass die versorgte Seite bei der Drehung entlastet oder leicht angehoben wird, um die Drehung zu erleichtern. Grundsätzlich ist es auch möglich, dass aus dem Stehen oder dem Stand heraus eine Rotation der kontralateralen, unversorgten Seite festgestellt wird, was in der rechten Darstellung gezeigt ist, woraus Rückschlüsse auf mögliche oder zukünftige Bewegungen in der versorgten Seite gezogen werden können. Ein Gehen im engeren Sinn findet nicht oder noch nicht statt, vielmehr eine Vorbereitung des Gehens oder eine Neuorientierung der Person und der orthopädietechnischen Einrichtung. Die Steuerung der orthopädietechnischen Einrichtung kann bei frühzeitiger Erkennung bereits an eine folgende Bewegung angepasst werden, beispielsweise um eine darauffolgende Schwungphaseneinleitung zu ermöglichen und entsprechend angepasst zu steuern.

Es ist auch möglich, dass bei Erkennung einer Rotation aus dem Stehen, aber auch aus der Standphase heraus, keine Einleitung oder Freischaltung einer Schwungphase stattfindet, um in solchen Situationen ein unerwartetes oder unkontrolliertes Einbeugen zu vermeiden. Auch kann bei einer Rotation das Umschalten in einen Sondermodus, zum Beispiel für das Treppensteigen, Hinsetzen oder Aufstehen, unterbunden oder abgebrochen werden, insbesondere um ein versehentliches Umschalten in einen solchen Sondermodus zu verhindern oder frühzeitig wieder in einen Ausgangsmodus zurück zu schalten. Entsprechend ist es vorteilhaft eine solche Rotation zu erkennen und die Steuerung anzupassen.

In der Figur 9 ist ein Beispiel gezeigt, wie die Orientierung der orthopädietechnischen Einrichtung 100 erfasst oder festgestellt werden kann. Die Orientierung in der Transversalebene kann sich beispielsweise auf eine externe Referenzgröße beziehen, beispielsweise ein Magnetfeld wie das Erd-Magnetfeld, wie in der linken Darstellung der Figur 9 beispielhaft anhand der Nord-Ausrichtung gezeigt. Die Orientierung der Längserstreckung beispielsweise des Fußes der orthopädietechnischen Einrichtung wird dann jeweils im Verhältnis zu der Nord-Südausrichtung des Magnetfeldes der Erde bestimmt. Dabei kann jedes geeignete Referenzsystem, das eine ausreichende Genauigkeit und Stabilität bietet, verwendet werden. Alternativ oder ergänzend ist es möglich, eine Rotation um einen bestimmten Winkel Δθ in Relation zu der kontralaterale Seite zu ermitteln und für die Steuerung heranzuziehen. Als Referenzgröße kann beispielsweise der Fuß, der Unterschenkel oder der Oberschenkel der kontralateralen Seite, beispielsweise einer unversorgten Seite oder einer ebenfalls versorgten, aber nicht hinsichtlich der Steuerung relevanten Seite, herangezogen werden. Die Orientierung der versorgten Seite relativ zu der kontralateralen Seite kann beispielsweise durch Sensoreinrichtungen festgestellt werden, die den Abstand, die Abstandsveränderungen und Bewegungen der kontralateralen Seite und ipsilateralen Seite zueinander erfassen. Es können Sender oder Markierungen, beispielsweise an dem Schuh oder dem Fußteil, an der kontralateralen Seite angeordnet sein, deren Orientierung relativ zu Bezugspunkten oder anderen Detektionselementen an der ipsilateralen Seite erfasst und ausgewertet werden. Durch Erfassung der Rotation von kontralateraler und ipsilateraler Seite, jeweils gegenüber einer gemeinsamen Referenz, kann die relative Rotation von ipsilateraler zu kontralateraler Seite bestimmt werden. Die relative Orientierung von ipsilateraler Seite zu kontralateraler Seite kann auch über zumindest zwei IMUs, beispielsweise eine auf der ipsilateralen Seite und eine auf der kontralateralen Seite angebrachte IMU und/oder einen oder mehrere Relativwinkelsensoren bestimmt werden.

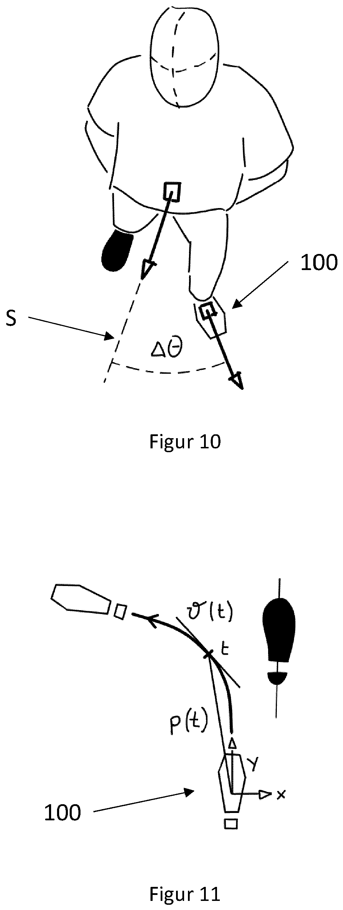

In der Figur 10 ist in einer schematischen Darstellung die Situation einer nutzenden Person einer orthopädietechnischen Einrichtung 100 gezeigt, bei der eine Drehung um die Längserstreckung der Längsachse der orthopädietechnischen Einrichtung 100, die beispielsweise im Wesentlichen in Gravitationsrichtung orientiert ist, durchgeführt worden ist. In der Figur 10 ist dargestellt, wie eine relative Rotation der versorgten Seite oder der orthopädietechnischen Einrichtung 100 relativ zu dem Rumpf der nutzenden Person ermittelt werden und für die Steuerung herangezogen werden kann. Die Sagittalebene S ist schematisch entlang der nach vorne gerichteten Orientierung dargestellt. Befindet sich die versorgte Seite oder orthopädietechnische Einrichtung 100 in einer Ausgangsstellung, bei der beispielsweise das Fußteil nach vorn orientiert und parallel zu der Sagittalebene S verläuft, kann die Rotation oder Drehung Δθ relativ zu dem Rumpf oder Torso der nutzenden Person ermittelt und zur Steuerung herangezogen werden. Die Ermittlung erfolgt beispielsweise über zumindest zwei IMUs oder zumindest einen Relativwinkelsensor.

Figur 11 zeigt die Bewegungsbahn einer Drehbewegung, bei der sich die versorgte Seite in der Schwungphase befindet. Ein Bewegungsmuster, wie es beispielsweise in der Figur 7 in der linken Darstellung gezeigt ist, kann hinsichtlich der Bewegungsbahn oder Trajektorie ermittelt werden. Die jeweilige Bahnkurve der ipsilateralen Seite, beispielsweise eines Fußteils, eines Unterschenkels oder eines Knies, wird beispielsweise über eine Wegintegration aus IMU-Daten ermittelt und für die Steuerung herangezogen. Die Bahnkurve oder Trajektorie ist dabei die Abfolge von Positionen p(t) zu aufeinanderfolgenden Zeitpunkten t. Insbesondere die Trajektorien in der Transversalebene bzw. die Projektion auf die Transversalebene gibt Aufschluss über eine Rotation um eine Längsachse. Die Trajektorie oder Bewegungsbahn kann als Absolutwert oder als ein Relativwert bezogen auf eine andere Komponente oder die kontralaterale Seite der Steuerung zugrunde gelegt werden. In Figur 11 ist die Position p des Fußes zu einem Zeitpunkt t in etwa in der Hälfte der Bewegung von einer Ausgangsposition rechts unten zu einer Endposition links oben, sowie die Bewegungsbahn in der Transversalebene als Abfolge von Positionen p(t) dargestellt. Die Position wird dabei relativ zur Ausgangsposition des ipsilateralen Fußes in einem kartesischen, stationären Koordinatensystem dargestellt. Neben der Position kann auch die momentane Bewegungsrichtung sowie deren Verlauf ermittelt werden. Bei einer Rotation ändert sich die Bewegungsrichtung in der Transversalebene im Zuge der Bewegung. In Figur 11 ist die momentane Bewegungsrichtung ϑ als Tangente auf die Bahnkurve zum Zeitpunkt t dargestellt. Die Bewegungsrichtung dreht sich in der Darstellung, ausgehend von einer Bewegung nach vorne, gegen den Uhrzeigersinn. Eine Drehung oder Änderung der Bewegungsrichtung kann ebenfalls als Rotation um eine Längsachse aufgefasst werden. Durch Erfassung der sich ändernden Bewegungsrichtung kann daher auf eine Rotation geschlossen und die Steuerung der Widerstände sowie Antriebe angepasst werden. Die Bewegungsrichtung kann dabei relativ, zum Beispiel gegenüber der kontralateralen Seite, aber auch absolut, gegenüber einem Referenzsystem bestimmt werden.

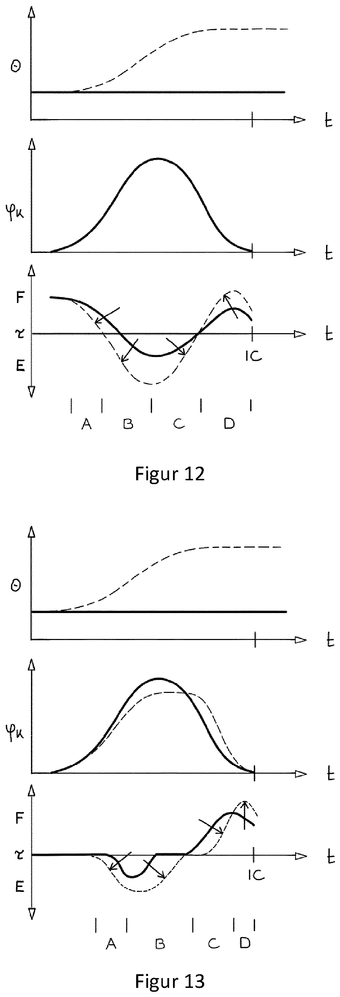

In der Figur 12 sind 3 verschiedene Parameter über die Zeit aufgetragen, nämlich die Orientierung θ, der Kniewinkel φₖ sowie das durch den Aktuator aufgebrachte, Kniemoment τ. In der Figur 12 sind mögliche Anpassungen in der Steuerung eines aktiven Prothesenkniegelenkes oder aktiven Orthesenkniegelenkes in der ipsilateralen Schwungphase, einschließlich der sogenannten Pre-Swing-Phase dargestellt. Die Orientierung Θ der ipsilateralen Seite beim Gehen geradeaus ist, mit der durchgezogenen Linie dargestellt. Die orthopädietechnische Einrichtung wird im Wesentlichen unverändert hinsichtlich der Orientierung um die Längsachse der Längserstreckung oder die Beinachse nach vorne geführt. Die orthopädietechnische Einrichtung bleibt in einer nahezu konstanten Orientierung, Θ, beispielsweise in der Sagittalebene S. Der gestrichelte Verlauf zeigt eine Rotation ausgehend von der Normalstellung oder Ausgangsstellung, bei der beispielsweise ein Fußteil nach vorne zeigt. Der Kniewinkelverlauf für das Gehen mit oder ohne Rotation um die Längserstreckung der Längsachse ist in dem dargestellten Ausführungsbeispiel in beiden Situationen gleich. Verändert wird das durch den Aktuator aufgebrachte Kniemoment τ, das einmal in Flexionsrichtung F oder in Extensionsrichtung E wirkt. Auch hier stellt die durchgezogene Linie den Verlauf beim geradeaus Gehen in der Ebene und die gestrichelte Linie den Verlauf bei einer durchgeführten Rotation dar. Ein Beugemoment oder Flexionsmoment in Flexionsrichtung F wird durch den Aktuator in der Pre-Swing-Phase beim Gehen geradeaus in der Ebene aufgebracht. Vor Erreichen des maximalen Kniewinkels φₖ wird die Flexionsunterstützung zunächst verringert und dann umgekehrt, um ein zu weites Einbeugen des Kniegelenkes zu vermeiden. Ein Extensionsmoment E wird aufgebracht, um eine Bewegungsumkehr einzuleiten oder zu unterstützen, sodass eine Streckung des Kniegelenkes in der Schwungphase stattfindet. Am Ende der Schwungphase wird wiederum ein Flexionsmoment F aufgebracht, um eine ungebremste Bewegung in den Extensionsanschlag zu verhindern. Das Flexionsmoment F wird nach Erreichen der vollständigen oder hinreichenden Streckung entweder minimiert oder reduziert, um eine Standphasenflexion einzuleiten oder zu ermöglichen. Bei der Rotation um die Längserstreckung der Längsachse kann in der Pre-Swing-Phase A ein Flexionsmoment früher verringert werden, sodass die Flexion weniger oder kürzer unterstützt wird oder früher ein Beugewiderstand bzw. ein Streckmoment aufgebracht wird (B). Das Streckmoment oder Extensionsmoment in der späten Schwungphase-Beugung B und/oder in der Schwungphase-Streckung C kann höher und/oder länger aufgebracht werden, ebenso kann ein erhöhter Streckwiderstand oder ein Flexionsmoment am Ende der Schwungphase D aufgebracht werden, wenn dies aufgrund der Rotation um die Längserstreckung der Längsachse notwendig ist, insbesondere um eine schnellere Streckung abzustoppen.

In der Figur 13 ist ein Parameterverlauf für die Steuerung eines passiven Kniegelenkes in der ipsilateralen Schwungphase einschließlich der Pre-Swing-Phase dargestellt, auch hier stellt eine durchgezogenen Linie das Gehen geradeaus und die gestrichelte Linie eine Rotation der ipsilateralen Seite dar. Anders als bei einer aktiven orthopädietechnischen Gelenkeinrichtung, beispielsweise einem motorisch angetriebenen künstlichen Kniegelenk, ändert sich bei einem passiven Kniegelenk der Kniewinkelverlauf dadurch, dass die maximale Einbeugung verringert und eine schnellere Extension oder Streckung erlaubt werden. Das Kniemoment, in diesem Fall ein durch einen Dämpfer oder eine Bremseinrichtung aufgebracht der Widerstand, wird bei einer erkannten Rotation in der Pre-Swing-Phase bzw. in der Schwungphasenflexion der Phase A früher einsetzen als beim Gehen geradeaus, ebenfalls ist ein höher Widerstand notwendig. Der Widerstand wird in der späten Schwungphasenflexion (Phase B) höher und länger anhaltend sein, in der Extensionsphase C später einsetzen und im Vergleich zum geradeaus Gehen geringer ausfallen. Dafür ist ein höherer Widerstand gegen eine Extension in der späten Schwungphase D kurz vor dem Aufsetzen des Fußes notwendig, um die Energie der vergleichsweise schnelleren Streckung zu dissipieren.

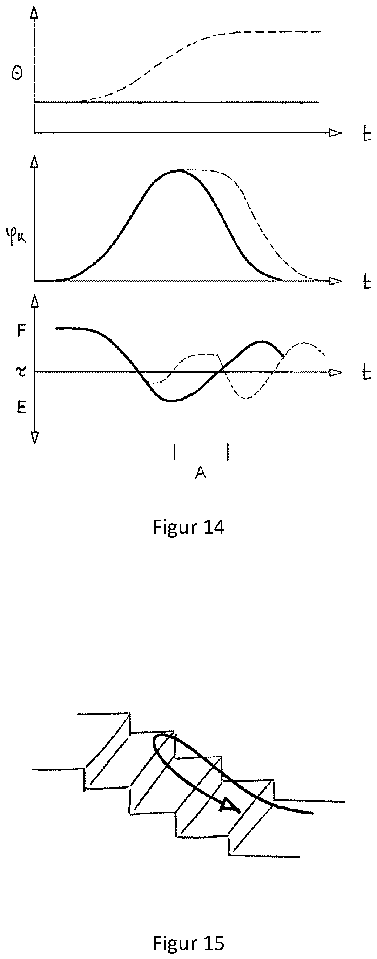

Eine weitere Variante ist in der Figur 14 dargestellt, bei der der Kniewinkel φₖ in der ipsilateralen Schwungphase bei einer Drehung länger gehalten wird. Dies kann beispielsweise bei einer langsamen Drehung von Vorteil sein, bei der ein eingebeugtes Kniegelenk länger in dieser Stellung gehalten wird. Alternativ zu einem Halten des Kniegelenkes in der Stellung kann auch eine verlangsamte Streckung und/oder Beugung erfolgen. Der Momentenverlauf des aufgebrachten Haltemomentes τ wird dann dergestalt geändert, dass nach Erreichen des maximalen Beugewinkels oder der maximalen Einbeugung ein Flexionsmoment erzeugt bzw. eine Extensionsmoment verringert wird, um das Kniegelenk in Beugung zu halten. Um eine Extension oder Bewegungsumkehr einzuleiten, wird dann ein Extensionsmoment aufgebracht oder ein Extensionswiderstand verringert, um eine Streckung des Kniegelenkes zu bewirken oder zu erleichtern.

Die Figur 15 zeigt einen Bewegungsablauf, bei der ein Sondermodus für die Steuerung der orthopädietechnischen Einrichtung, beispielsweise eines Prothesenkniegelenkes oder Orthesenkniegelenkes, verändert wird. Wird erkannt, dass im Treppensteigmodus eine Rotation der orthopädietechnischen Einrichtung um die Längserstreckung der Längsachse der orthopädietechnischen Einrichtung und/oder einer kontralateralen Seite auftritt, kann ein anderer Gehmodus eingestellt werden. Wird die Drehung auf einer Treppe erkannt, zum Beispiel dadurch, dass kein Anheben des Fußes der ipsilateralen Seite und eine Rotation stattfindet, wird der Treppensteigmodus verlassen und beispielsweise in einen Grundmodus, den Modus für das Gehen in der Ebene oder in einen Modus für das Treppen abwärts Gehen gewechselt. Eine bereits eingeleitete Bewegung für das nach Oben-Setzen des Fußes im Treppenmodus, zum Beispiel eine Knieflexion, wird bei einer erkannten Rotation abgebrochen und das Bein wieder gestreckt um das Aufsetzen der versorgten Seite und die Lastübernahme auf diese zu ermöglichen.

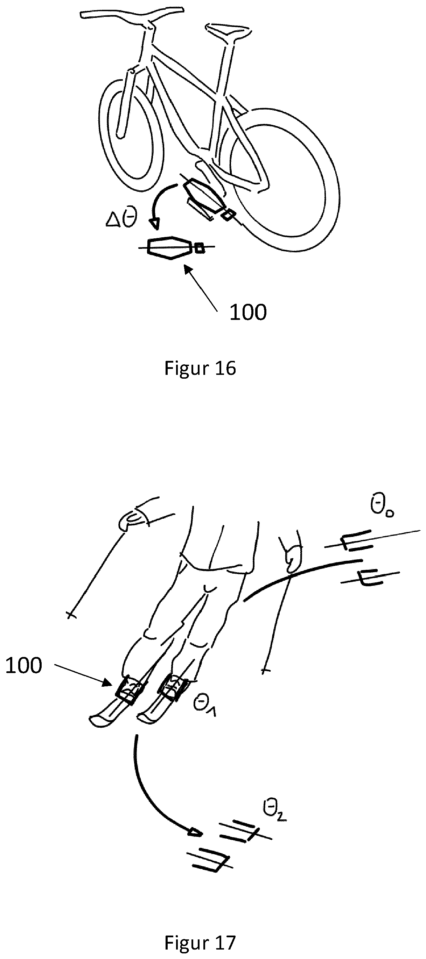

In der Figur 16 ist eine Variante gezeigt, bei der ein Sondermodus, in dem dargestellten Beispiel für das Fahrradfahren, deaktiviert wird. Wird das Absteigen von einem Fahrrad aufgrund einer Bewegung der versorgten, ipsilateralen Seite erkannt, beispielsweise über eine schnelle Drehung des Prothesenfußes oder des Fußteils einer Orthese, wird der Fahrradfahrmodus verlassen. Die Drehung kann beispielsweise aus dem Lösen einer Verriegelung des Fußes aus einer Pedale folgen. Das Auslösen einer Deaktivierung des Sondermodus kann in Abhängigkeit von dem Erreichen eines Schwellwertes erfolgen. Die Drehung des Fußes muss eine gewisse Geschwindigkeit oder einen relativen Verdrehwinkel überschreiten, bevor der Sondermodus deaktiviert wird, da während eines normalen Bewegungsablaufes in dem Sondermodus ebenfalls Drehbewegungen, insbesondere langsame Drehbewegungen, auftreten können.

Ein weiteres Ausführungsbeispiel eines Bewegungsablaufes mit einer gesonderten Steuerung für einen Aktuator ist in der Figur 17 dargestellt, bei der das Skifahren gezeigt ist. Erkennt die Steuerung das Skifahren oder wird eine solche Sonderfunktion durch die nutzende Person aktiviert, wird in Abhängigkeit von der Orientierung oder einer Orientierungsänderung die Steuerung angepasst. Beispielsweise ist bei einem sogenannten Talski bei einer Seitwärtsneigung nach innen und damit zum Hang zunächst eine Extension gesperrt. Dies ermöglicht eine besonders gute Kraftübertragung. Wird dann bei einem Wechsel aufgrund eines Schwunges eine Außenrotation des Talskis erkannt, wird die Extensionssperre gelöst und eine Streckung kann erfolgen. Gleichermaßen kann bei einem Wechsel von Bergski zu Talski eine Sperre aktiviert werden.

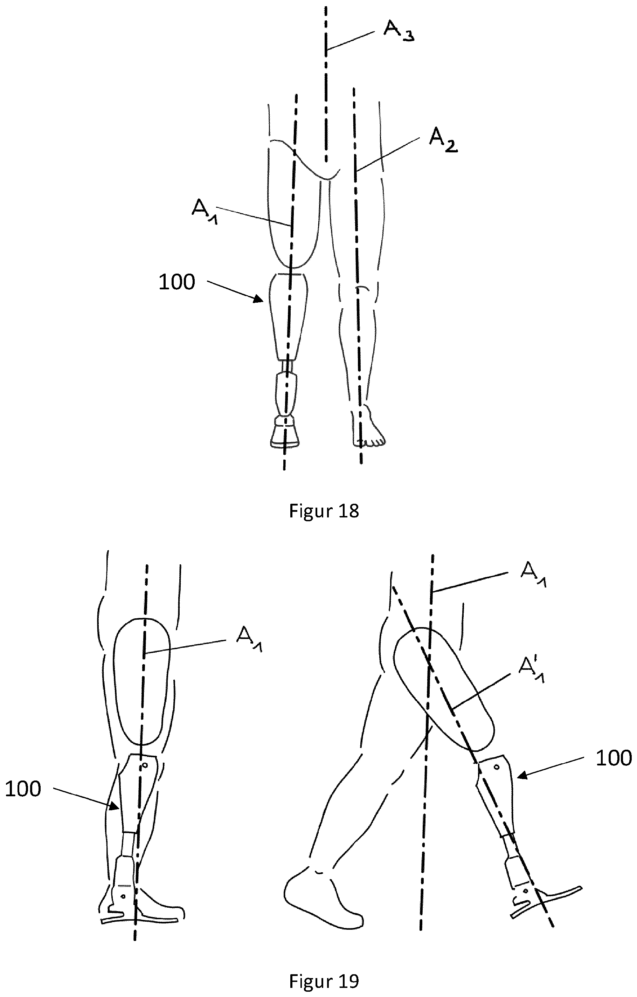

Figur 18 zeigt unterschiedliche Längsachsen in der Frontalebene beim Stehen, um die eine Rotation stattfinden kann. A1 ist die Längsachse der orthopädietechnischen Einrichtung 100. A2 ist die Längsachse der kontralateralen Seite, die in diesem Fall nicht mit einem Hilfsmittel versorgt ist. A3 ist die proximal-distal-Achse des Rumpfes.

Figur 19 zeigt links die Längsachse A1 der orthopädietechnischen Einrichtung 100 im Stehen. Wird die orthopädietechnische Einrichtung 100 im Zuge einer Bewegung verschwenkt, was rechts in der Figur 19 zeigt ist, ändert sich auch die Orientierung der Längsachse A1 in die Längsachse A1' der orthopädietechnischen Einrichtung 100 in der verschwenkten Stellung. Eine Drehbewegung zeigt in dieser Darstellung aus der Zeichenebene heraus oder hinein und ist daher nicht ersichtlich. Eine Rotation um eine Längsachse kann sich nun sowohl auf die momentane Längserstreckung A1'der orthopädietechnischen Einrichtung 100 beziehen, die in der Sagittalebene mitrotiert, als auch auf die Längserstreckung A1 in einer Referenzposition, wie beispielsweise in der links dargestellten Position im Stehen. Gleiches trifft auf die Längserstreckung anderer Komponenten und Körperteile zu, wie dem kontralateralen Bein oder dem Rumpf.

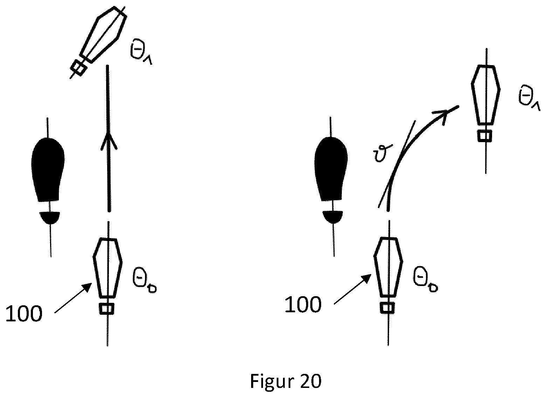

Eine Rotation kann sowohl eine Verdrehung einer Komponente um eine Längsachse sein als auch eine gekrümmte Bewegungsbahn, in der die Bewegungsrichtung mit der Bewegung rotiert. In vorangegangenen Figuren wurden diese Bewegungen zumeist in überlagerter Form dargestellt. Diese zwei Formen der Rotation können jedoch auch isoliert erfolgen. Figur 20 zeigt links eine Bewegung, in der der Fuß der orthopädietechnischen Einrichtung 100 geradlinig aus einer Schrittstellung nach vorne gesetzt wird und dabei von einer parallelen Stellung θ₀ im Uhrzeigersinn nach außen rotiert (θ₁). Die Rotation findet um die Längsachse der ipsilateralen Seite statt. In der rechten Darstellung wird der Fuß der orthopädietechnischen Einrichtung 100 ausgehend von einer Schrittstellung, in der der sich der Fuß der versorgten Seite hinter dem kontralateralen Fuß befindet, schräg nach außen gesetzt. Der Fuß der versorgten Seite beschreibt eine Kreisbahn. Die Orientierungen des Fußes in der Ausgangsstellung θ₀ und der Endstellung θ₁ sind identisch. Die Änderung der Bewegungsrichtung ϑ aufgrund der kreisabschnittsförmigen, beziehungsweise krummlinigen Bewegung ist dabei im Sinne der Erfindung eine Rotation um das kontralaterale Standbein. In vielen Fällen tritt eine Kombination aus einer Verdrehung und einer krummlinigen Bewegungsbahn auf, wie zum Beispiel in Figur 6 gezeigt.

## Patentansprüche

1. Verfahren zur Steuerung einer orthopädietechnischen Einrichtung (100) der unteren Extremität mit einem proximalen Oberteil (2) und einem distalen Unterteil (3), die gelenkig um zumindest eine Schwenkachse (4) miteinander unter Ausbildung eines Gelenkes (5) aneinander gelagert sind, mit Einrichtungen zum Festlegen der orthopädietechnischen Einrichtung an eine Gliedmaße und zumindest einem Aktuator (6), der mit einer Steuerungseinrichtung (7) gekoppelt ist, die auf der Grundlage von Sensordaten zumindest eines mit der Steuerungseinrichtung (7) gekoppelten Sensors (8) den Aktuator (6) aktiviert oder deaktiviert, um einen Verschwenkungswiderstand und/oder eine Relativbewegung des Oberteils (2) zu dem Unterteil (3) oder zweier Bauteile der orthopädietechnischen Einrichtung zueinander zu beeinflussen, wobei über die Sensordaten eine Orientierung und/oder Orientierungsänderung um die Längsachse der Längserstreckung in proximal-distal-Richtung der orthopädietechnischen Einrichtung (100) und/oder einer kontralateralen Gliedmaße erfasst werden und auf der Grundlage der Orientierung und/oder Orientierungsänderung um die Längsachse der Aktuator (6) aktiviert, deaktiviert oder ein Sollwert für den Aktuator (6) moduliert wird, **dadurch gekennzeichnet, dass** bei einer Rotation mit der versorgten Seite um die Längsachse der Längserstreckung der versorgten Seite in der Standphase der Flexionswiderstand im Kniegelenk nicht oder in einem geringeren Maß als beim Geradeausgehen reduziert wird und/oder keine oder eine geringere Flexionsbewegung als beim Geradeausgehen eingeleitet wird.

2. Verfahren nach Anspruch 1, **dadurch gekennzeichnet, dass** während der Nutzung der orthopädietechnischen Einrichtung (100) im angelegten Zustand die Sensordaten ermittelt werden und der Aktuator (6) aktiviert, deaktiviert oder der Sollwert für den Aktuator (6) moduliert wird.

3. Verfahren nach Anspruch 1 oder 2, **dadurch gekennzeichnet, dass** die Orientierung oder Verlagerung über einen Raumlagesensor, eine IMU und/oder Winkelsensoren erfasst und ermittelt werden.

4. Verfahren nach einem der voranstehenden Ansprüche, **dadurch gekennzeichnet, dass** Kräfte, Momente und/oder Beschleunigungen über Sensoren (8) erfasst und der Steuerung zugrunde gelegt werden.

5. Verfahren nach einem der voranstehenden Ansprüche, **dadurch gekennzeichnet, dass** die orthopädietechnische Einrichtung (100) als Prothese oder Orthese ausgebildet ist und ein künstliches Kniegelenk (5) und/oder ein künstliches Knöchelgelenk (5) aufweist, denen der Aktuator (6) zugeordnet ist.

6. Verfahren nach einem der voranstehenden Ansprüche, **dadurch gekennzeichnet, dass** der zumindest eine Sensor (8) an der orthopädietechnischen Einrichtung (100), der kontralateralen Gliedmaße oder dem Rumpf des Nutzers angeordnet wird oder dass mehrere Sensoren (8) an der orthopädietechnischen Einrichtung (100), der kontralateralen Gliedmaße oder dem Rumpf des Nutzers angeordnet werden und die Sensordaten der Steuerung zugrunde gelegt werden.

7. Verfahren nach einem der voranstehenden Ansprüche, **dadurch gekennzeichnet, dass** bei einer Rotation der versorgten Seite um die Längsachse der Längserstreckung der kontralateralen Gliedmaße in der Schwungphase der orthopädietechnischen Einrichtung eine Vergrößerung eines Flexionswiderstandes eingeleitet wird und/oder eine Verringerung eines Extensionswiderstandes oder eine Extension eingeleitet wird.

8. Verfahren nach einem der voranstehenden Ansprüche, **dadurch gekennzeichnet, dass** bei einer Rotation der versorgten Seite um die Längsachse der Längserstreckung der kontralateralen Gliedmaße in der Schwungphase der orthopädietechnischen Einrichtung eine Hüftbeugung unterstützt wird.

9. Verfahren nach einem der voranstehenden Ansprüche, **dadurch gekennzeichnet, dass** bei einer Rotation der versorgten Seite um die Längsachse der Längserstreckung der versorgten Seite in der Standphase der Flexionswiderstand erhöht, eine Einbeugung verringert und/oder eine Einbeugung verhindert wird.

10. Verfahren nach einem der voranstehenden Ansprüche, **dadurch gekennzeichnet, dass** die Orientierung und/oder Orientierungsänderung um die Längsachse der Längserstreckung in proximal-distal-Richtung der orthopädietechnischen Einrichtung (100) und/oder einer kontralateralen Gliedmaße relativ zu dem Rumpf der Patienten oder zu einer externen Bezugsorientierung erfasst werden.

11. Verfahren nach einem der voranstehenden Ansprüche, **dadurch gekennzeichnet, dass** der Aktuator in Abhängigkeit von der Dauer, dem Ausmaß, der Geschwindigkeit und/oder des Geschwindigkeitsprofils der Orientierungsänderung und/oder einer Bewegung aktiviert oder deaktiviert wird oder ein Sollwert für den Aktuator (6) moduliert wird.

12. Verfahren nach einem der voranstehenden Ansprüche, **dadurch gekennzeichnet, dass** eine IMU als Sensor an der versorgten Gliedmaße, der kontralateralen Gliedmaße und/oder dem Rumpf des Patienten angebracht wird und eine relative Rotation der Gliedmaße oder Gliedmaßen zu dem Rumpf erfasst und der Aktivierung oder Deaktivierung oder Modulierung eines Sollwertes des Aktuators zugrunde gelegt wird.

13. Verfahren nach einem der voranstehenden Ansprüche, **dadurch gekennzeichnet, dass** die Bewegungsbahn in der Transversalebene erfasst und der Aktivierung oder Deaktivierung oder Modulierung eines Sollwertes des Aktuators zugrunde gelegt wird.

14. Verfahren nach einem der voranstehenden Ansprüche, **dadurch gekennzeichnet, dass** bei einer Rotation der versorgten Seite um die Längsachse der Längserstreckung der versorgten Seite ein Sondermodus verlassen und/oder nicht aktiviert wird.

15. Orthopädietechnische Einrichtung (100) der unteren Extremität mit einem Oberteil (2) und einem Unterteil (3), die gelenkig um zumindest eine Schwenkachse (4) miteinander unter Ausbildung eines Gelenkes (5) aneinander gelagert sind und zumindest einem Aktuator (6), der mit einer Steuerungseinrichtung (7) gekoppelt ist, die auf der Grundlage von Sensordaten zumindest eines mit der Steuerungseinrichtung (7) gekoppelten Sensors (8) den Aktuator (6) aktiviert oder deaktiviert, um einen Verschwenkungswiderstand oder eine Relativbewegung des Oberteils (2) zu dem Unterteil (3) zu beeinflussen, **dadurch gekennzeichnet, dass** der zumindest eine Sensor (8) ausgebildet und eingerichtet ist, Sensordaten über eine Orientierung und/oder Veränderung der Orientierung der orthopädietechnischen Einrichtung (100) um die Längsachse der Längserstreckung in proximal-distal-Richtung der orthopädietechnischen Einrichtung (100) und/oder einer kontralateralen Gliedmaße erfasst werden und dass die Steuerungseinrichtung (7) eingerichtet ist, auf der Grundlage der Orientierung und/oder Orientierungsänderung um die Längsachse den Aktuator (6) zu aktivieren, zu deaktivieren oder einen Sollwert für den Aktuator (6) zu modulieren und die Steuerungseinrichtung (7) eingerichtet ist, bei einer Rotation mit der versorgten Seite um die Längsachse der Längserstreckung der versorgten Seite in der Standphase den Flexionswiderstand im Kniegelenk nicht oder in einem geringeren Maß als beim Geradeausgehen zu reduzieren und/oder keine oder eine geringere Flexionsbewegung als beim Geradeausgehen einzuleiten.

16. Orthopädietechnische Einrichtung (100) nach Anspruch 15, **dadurch gekennzeichnet, dass** zumindest ein Sensor (7) als IMU ausgebildet und an dem Oberteil (2) oder dem Unterteil (3), der versorgten oder unversorgten kontralateralen Gliedmaße oder dem Rumpf des Patienten befestigt und mit der Steuerungseinrichtung (7) gekoppelt ist.

17. Orthopädietechnische Einrichtung (100) nach Anspruch 15 oder 16, **dadurch gekennzeichnet, dass** zumindest ein Kraftsensor, Beschleunigungssensor, Winkelsensor und/oder Momentensensor an dem Oberteil (2) und/oder dem Unterteil (3) angeordnet ist.

## Claims

1. A method for controlling a lower-limb orthopedic device (100) having a proximal upper part (2) and a distal lower part (3), which are mounted on each other in an articulated manner about at least one pivot axis (4) so as to form a joint (5) with each other, means for fastening the orthopedic device to a limb, and at least one actuator (6) coupled to a control device (7) which activates or deactivates the actuator (6) on the basis of sensor data from at least one sensor (8) coupled to the control device (7), in order to influence a pivoting resistance and/or a movement of the upper part (2) relative to the lower part (3) or of two components of the orthopedic device relative to each other, wherein an orientation and/or change in orientation about the longitudinal axis of the longitudinal extent in the proximal-distal direction of the orthopedic device (100) and/or of a contralateral limb are detected using the sensor data, and the actuator (6) is activated or deactivated, or a setpoint value for the actuator (6) is modulated, on the basis of the orientation and/or change in orientation about the longitudinal axis, **characterized in that**, during a rotation with the treated side about the longitudinal axis of the longitudinal extent of the treated side in the stance phase, the flexion resistance in the knee joint is not reduced or is reduced to a smaller extent than when walking straight ahead and/or no flexion movement or a smaller flexion movement than when walking straight ahead is initiated..

2. The method as claimed in claim 1, **characterized in that** the sensor data are determined and the actuator (6) is activated or deactivated, or the setpoint value for the actuator (6) is modulated, during the use of the orthopedic device (100) in the fitted state.

3. The method as claimed in claim 1 or 2, **characterized in that** the orientation or displacement is detected and determined via a spatial position sensor, an IMU and/or angle sensors.

4. The method as claimed in any one of the preceding claims, **characterized in that** forces, moments and/or accelerations are detected via sensors (8) and used as the basis for the control.

5. The method as claimed in any one of the preceding claims, **characterized in that** the orthopedic device (100) is designed as a prosthesis or orthosis and has an artificial knee joint (5) and/or an artificial ankle joint (5) to which the actuator (6) is assigned.

6. The method as claimed in any one of the preceding claims, **characterized in that** the at least one sensor (8) is arranged on the orthopedic device (100), the contralateral limb or the torso of the user, or **in that** several sensors (8) are arranged on the orthopedic device (100), the contralateral limb or the torso of the user and the sensor data are used as the basis for the control.

7. The method as claimed in any one of the preceding claims, **characterized in that**, during a rotation of the treated side about the longitudinal axis of the longitudinal extent of the contralateral limb in the swing phase of the orthopedic device, an increase in flexion resistance is initiated and/or a reduction in extension resistance or an extension is initiated.

8. The method as claimed in any one of the preceding claims, **characterized in that** hip flexion is supported during a rotation of the treated side about the longitudinal axis of the longitudinal extent of the contralateral limb in the swing phase of the orthopedic device.

9. The method as claimed in any one of the preceding claims, **characterized in that**, during a rotation of the treated side about the longitudinal axis of the longitudinal extent of the treated side in the stance phase, the flexion resistance is increased, bending is reduced and/or bending is prevented.

10. The method as claimed in any one of the preceding claims, **characterized in that** the orientation and/or change in orientation about the longitudinal axis of the longitudinal extent in the proximal-distal direction of the orthopedic device (100) and/or of a contralateral limb relative to the torso of the patient or to an external reference orientation are detected.

11. The method as claimed in any one of the preceding claims, **characterized in that** the actuator is activated or deactivated, or a setpoint value for the actuator (6) is modulated, depending on the duration, extent, speed and/or speed profile of the change in orientation and/or of a movement.

12. The method as claimed in any one of the preceding claims, **characterized in that** an IMU is attached as sensor to the treated limb, the contralateral limb and/or the torso of the patient, and a rotation of the limb or limbs relative to the torso is detected and used as the basis for the activation or deactivation or modulation of a setpoint value of the actuator.

13. The method as claimed in any one of the preceding claims, **characterized in that** the trajectory in the transverse plane is detected and used as the basis for the activation or deactivation or modulation of a setpoint value of the actuator.

14. The method as claimed in any one of the preceding claims, **characterized in that** a special mode is exited and/or not activated during a rotation of the treated side about the longitudinal axis of the longitudinal extent of the treated side.

15. A lower-limb orthopedic device (100) having an upper part (2) and a lower part (3), which are mounted on each other in an articulated manner about at least one pivot axis (4) so as to form a joint (5) with each other, and at least one actuator (6) coupled to a control device (7) which activates or deactivates the actuator (6) on the basis of sensor data from at least one sensor (8) coupled to the control device (7), in order to influence a pivoting resistance or a movement of the upper part (2) relative to the lower part (3), **characterized in that** the at least one sensor (8) is designed and configured to detect sensor data concerning an orientation and/or change in orientation of the orthopedic device (100) about the longitudinal axis of the longitudinal extent in the proximal-distal direction of the orthopedic device (100) and/or of a contralateral limb, and **in that** the control device (7) is configured to activate or deactivate the actuator (6), or to modify a setpoint value for the actuator (6), on the basis of the orientation and/or change in orientation about the longitudinal axis and that the control device (7) is configured to initiate that, during a rotation with the treated side about the longitudinal axis of the longitudinal extent of the treated side in the stance phase, the flexion resistance in the knee joint is not reduced or is reduced to a smaller extent than when walking straight ahead and/or no flexion movement or a smaller flexion movement than when walking straight ahead

16. The orthopedic device (100) as claimed in claim 15, **characterized in that** at least one sensor (7) is designed as an IMU and is fastened to the upper part (2) or the lower part (3), the treated or untreated contralateral limb or the torso of the patient and is coupled to the control device (7).

17. The orthopedic device (100) as claimed in claim 15 or 16, **characterized in that** at least one force sensor, acceleration sensor, angle sensor and/or moment sensor is arranged on the upper part (2) and/or the lower part (3).

## Revendications

1. Procédé de commande d'un dispositif orthopédique (100) destiné aux membres inférieurs, comprenant une partie supérieure (2) proximale et une partie inférieure (3) distale qui sont montées l'une sur l'autre de manière articulée autour d'au moins un axe de pivotement (4) en formant une articulation (5), comprenant des moyens destinés à fixer le dispositif orthopédique à un membre, et au moins un actionneur (6) couplé à un dispositif de commande (7) qui, sur la base des données de capteur fournies par au moins un capteur (8) couplé au dispositif de commande (7), active ou désactive l'actionneur (6) afin d'influencer une résistance au pivotement et/ou un mouvement relatif de la partie supérieure (2) par rapport à la partie inférieure (3) ou de deux composants du dispositif orthopédique l'un par rapport à l'autre, les données de capteur permettant de détecter une orientation et/ou un changement de l'orientation autour de l'axe longitudinal de l'étendue longitudinale dans la direction proximale-distale du dispositif orthopédique (100) et/ou d'un membre controlatéral, et, sur la base de l'orientation et/ou du changement de l'orientation autour de l'axe longitudinal, l'actionneur (6) est activé ou désactivé ou une valeur de consigne pour l'actionneur (6) est modulée,
**caractérisé en ce que**, lors d'une rotation du côté traité autour de l'axe longitudinal de l'étendue longitudinale dudit côté traité, pendant la phase d'appui, la résistance à la flexion dans l'articulation du genou n'est pas réduite, ou l'est dans une moindre mesure que lors d'une marche en ligne droite, et/ou aucun mouvement de flexion n'est initié ou un mouvement de flexion moindre que lors de la marche en ligne droite est initié.

2. Procédé selon la revendication 1,
**caractérisé en ce que**, pendant l'utilisation du dispositif orthopédique (100) à l'état appliqué, les données de capteur sont enregistrées et l'actionneur (6) est activé ou désactivé ou la valeur de consigne de l'actionneur (6) est modulée.

3. Procédé selon la revendication 1 ou 2,
**caractérisé en ce que** l'orientation ou le déplacement sont détectés et déterminés à l'aide d'un capteur de position spatiale, d'une unité de mesure inertielle et/ou de capteurs d'angule.

4. Procédé selon l'une des revendications précédentes,
**caractérisé en ce que** les forces, les couples et/ou les accélérations sont détectés par des capteurs (8) et servent de base à la commande.

5. Procédé selon l'une des revendications précédentes,
**caractérisé en ce que** le dispositif orthopédique (100) est conçu sous la forme d'une prothèse ou d'une orthèse et comporte une articulation artificielle du genou (5) et/ou une articulation artificielle de la cheville (5) auxquelles est associé l'actionneur (6).

6. Procédé selon l'une des revendications précédentes,
**caractérisé en ce que** ledit au moins un capteur (8) est disposé sur le dispositif orthopédique (100), sur le membre controlatéral ou sur le tronc de l'utilisateur, ou **en ce que** plusieurs capteurs (8) sont disposés sur le dispositif orthopédique (100), sur le membre controlatéral ou sur le tronc de l'utilisateur, et les données de capteur servent de base à la commande.

7. Procédé selon l'une des revendications précédentes,
**caractérisé en ce que**, lors d'une rotation du côté traité autour de l'axe longitudinal de l'étendue longitudinale du membre controlatéral, pendant la phase pendulaire du dispositif orthopédique, une augmentation de la résistance à la flexion est initiée et/ou une diminution d'une résistance à l'extension ou une extension est initiée.

8. Procédé selon l'une des revendications précédentes,
**caractérisé en ce que**, lors d'une rotation du côté traité autour de l'axe longitudinal de l'étendue longitudinale du membre controlatéral, pendant la phase pendulaire du dispositif orthopédique, une flexion de la hanche est favorisée.

9. Procédé selon l'une des revendications précédentes,
**caractérisé en ce que**, lors d'une rotation du côté traité autour de l'axe longitudinal de l'étendue longitudinale dudit côté traité, pendant la phase d'appui, la résistance à la flexion est augmentée, une flexion est réduite et/ou une flexion est empêchée.

10. Procédé selon l'une des revendications précédentes,
**caractérisé en ce que** l'orientation et/ou le changement de l'orientation autour de l'axe longitudinal de l'étendue longitudinale dans la direction proximale-distale du dispositif orthopédique (100) et/ou d'un membre controlatéral sont détectés par rapport au tronc du patient ou à une orientation de référence externe.

11. Procédé selon l'une des revendications précédentes,
**caractérisé en ce que** l'actionneur est activé ou désactivé ou une valeur de consigne pour l'actionneur (6) est modulée en fonction de la durée, de l'amplitude, de la vitesse et/ou du profil de vitesse du changement de l'orientation et/ou d'un mouvement.

12. Procédé selon l'une des revendications précédentes,
**caractérisé en ce qu'**une unité de mesure inertielle est fixée en tant que capteur sur le membre traité, sur le membre controlatéral et/ou sur le tronc du patient, et une rotation relative du membre ou des membres par rapport au tronc est détectée, laquelle sert de base à l'activation ou à la désactivation ou à la modulation d'une valeur de consigne de l'actionneur.

13. Procédé selon l'une des revendications précédentes,
**caractérisé en ce que** la trajectoire de mouvement dans le plan transversal est détectée et sert de base à l'activation ou à la désactivation ou à la modulation d'une valeur de consigne de l'actionneur.

14. Procédé selon l'une des revendications précédentes,
**caractérisé en ce que**, lors d'une rotation du côté traité autour de l'axe longitudinal de l'étendue longitudinale dudit côté traité, un mode spécial est quitté et/ou n'est pas activé.

15. Dispositif orthopédique (100) destiné aux membres inférieurs, comprenant une partie supérieure (2) et une partie inférieure (3) qui sont montées l'une sur l'autre de manière articulée autour d'au moins un axe de pivotement (4) en formant une articulation (5), et comprenant au moins un actionneur (6) couplé à un dispositif de commande (7) qui, sur la base des données de capteur fournies par au moins un capteur (8) couplé au dispositif de commande (7), active ou désactive l'actionneur (6) afin d'influencer une résistance au pivotement ou un mouvement relatif de la partie supérieure (2) par rapport à la partie inférieure (3),
**caractérisé en ce que** ledit au moins un capteur (8) est conçu et agencé pour enregistrer des données de capteur concernant une orientation et/ou un changement de l'orientation du dispositif orthopédique (100) autour de l'axe longitudinal de l'étendue longitudinale dans la direction proximale-distale du dispositif orthopédique (100) et/ou d'un membre controlatéral, et **en ce que** le dispositif de commande (7) est agencé pour, sur la base de l'orientation et/ou du changement de l'orientation autour de l'axe longitudinal, activer ou désactiver l'actionneur (6) ou moduler une valeur de consigne pour l'actionneur (6), et le dispositif de commande (7) est agencé pour, lors d'une rotation du côté traité autour de l'axe longitudinal de l'étendue longitudinale dudit côté traité, pendant la phase d'appui, ne pas réduire la résistance à la flexion dans l'articulation du genou, ou pour la réduire dans une moindre mesure que lors de la marche en ligne droite, et/ou pour ne pas initier de mouvement de flexion, ou pour initier un mouvement de flexion moindre que lors de la marche en ligne droite.

16. Dispositif orthopédique (100) selon la revendication 15,
**caractérisé en ce qu'**au moins un capteur (7) est conçu comme une unité de mesure inertielle et est fixé à la partie supérieure (2) ou à la partie inférieure (3), au membre controlatéral traité ou non traité ou au tronc du patient, et est couplé au dispositif de commande (7).

17. Dispositif orthopédique (100) selon la revendication 15 ou 16, **caractérisé en ce qu'**au moins un capteur de force, un capteur d'accélération, un capteur d'angle et/ou un capteur de couple est disposé sur la partie supérieure (2) et/ou sur la partie inférieure (3).
